(19) Europäisches Patentamt

European Patent Office

Office européen des brevets

(11) **EP 2 974 648 A1**

(12) **EUROPEAN PATENT APPLICATION**

(43) Date of publication:
20.01.2016 Bulletin 2016/03

(51) Int Cl.:
*A61B 5/00* (2006.01)     *A61B 5/08* (2006.01)
*A61B 5/113* (2006.01)

(21) Application number: 14198066.4

(22) Date of filing: 15.12.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 18.12.2013 US 201361917790 P

(71) Applicant: **Analog Devices Global Hamilton (BM)**

(72) Inventors:
• **O'Dwyer, Thomas G.**
  **Clonlara (IE)**
• **O'Connor, John P.**
  **Bradford, MA Massachusetts 01825 (US)**
• **Lukashevich, Dzianis**
  **80993 Munich Bavaria (DE)**

(74) Representative: **Thompson, Andrew John et al Withers & Rogers LLP 4 More London Riverside London SE1 2AU (GB)**

(54) **System and method for measuring respiration with accelerometers**

(57) The respiration rate of a patient may be measured by sensing respiration motion using two MEMS devices, and by processing a respiration signal produced by processing the outputs of the two MEMS devices. Some embodiments dispose two accelerometers around a patient's abdomen and determine respiratory motion from the difference between the outputs of the two accelerometers. Other embodiments dispose two non-identical accelerometers at substantially the same location on the patient's body, such that each of the accelerometers is exposed to the same respiratory motion, and determine respiratory motion from the difference between the outputs of the two accelerometers.

Fig. 5A

**Description**

**Technical Field**

The present inventions relate to measurement of respiration in a patient, and more particularly to measurement of respiration using accelerometers.

**Background Art**

It is known in the prior art to measure the respiration of a patient, including the use of breathing straps having sensors that change impedance with the expansion and contraction of the patient's chest, and via infrared thermal imaging, for example. A direct measurement of respiration may be obtained by the use of a spirometer.
Human respiration rate has also been measured by using motion sensors (e.g., accelerometers) to sense the change in angle of a patient's chest wall. However, a problem in using motion sensors to detect respiration is that motion of a patient's chest wall due to respiration may be small relative to the motion of the patient's body, for example if the patient is walking, because a motion sensor disposed to sense a motion of a patient's chest also senses other motions of the patient's body. A chest motion signal due to the patient's respiration may be small relative to a signal due to such other motions, thus making it difficult to distinguish the small respiration signal from the larger motion signal.

**Summary of the Embodiments**

A first embodiment provides a method of detecting the respiration of a subject, the subject having a thorax, an abdomen, and a spine having a lower spinal region disposed between the coccyx and the thorax, including the steps of affixing a first accelerometer to the subject's lower spinal region, the first accelerometer having a first axis of sensitivity substantially normal to a portion of the subject's back, and configured to produce a first motion signal indicative of motion along the first axis of sensitivity; affixing a second accelerometer to an anterior portion of the subject's lower abdominal region, the second accelerometer having a second axis of sensitivity substantially parallel to the first axis of sensitivity, and configured to produce a second motion signal indicative of motion along the second axis of sensitivity, the first accelerometer and the second accelerometer disposed such that the subject's abdomen is between the first accelerometer and the second accelerometer, and the first axis of sensitivity and the second axis of sensitivity pass through the subject's abdomen; and processing (e.g., in a circuit) the second motion signal and the first motion signal to produce a respiration signal, the respiration signal indicative of the motion of the subject's abdomen in response to the subject's respiration. The method may also include determining, for example using the circuit, the number of cycles of respiration that occurred over a predefined period of time.
In some embodiments, the first accelerometer and the second accelerometer are disposed so as to produce a first motion signal that is complementary to the second motion signal in response to a commonly applied gross motion. Further, the step of determining the difference between the second motion signal and the first motion signal includes summing the first motion signal and the second motion signal, for example in a summing circuit.
In another embodiment, a method of detecting the respiration of a patient includes providing a first accelerometer affixed to the subject, the first accelerometer having a first response characteristic configured to produce a first output signal in response to both a respiration motion of the patient and a gross motion of the patient; and providing a second accelerometer affixed to the subject, the second accelerometer having a second response characteristic configured to produce a second output signal in response to the gross motion of the patient, but not in response to the respiration motion of the patient. The method then uses a circuit for acquiring, from the first accelerometer, the first motion output signal; acquiring, from the second accelerometer, the second motion output signal; and determining the difference between the second motion signal and the first motion signal to produce a cyclical respiration signal, the respiration signal produced in response to the subject's respiration.
The accelerometers may be arranged in a variety of configurations. For example, some embodiments include affixing the first accelerometer and the second accelerometer to the patient such that the first accelerometer and the second accelerometer are within 3 centimeters of one another. In some embodiments, the first accelerometer and the second accelerometer are in a stacked configuration.
In some embodiments, the first response characteristic includes a dynamic range with a noise floor sufficiently low such that the first accelerometer is configured to produce a first output signal in response to both a respiration motion of the patient and a gross motion of the patient; and the second response characteristic includes a dynamic range with a low-end that is above the amplitude of the respiration motion of the patient but below the amplitude of a gross motion of the patient, such that the second accelerometer is configured to produce a second output signal in response to the gross motion of the patient, but not in response to the respiration motion of the patient.
In another embodiment, the first response characteristic includes a low-frequency cutoff at a frequency sufficiently low such that the first accelerometer is configured to produce a first output signal in response to both a respiration motion of the patient and a gross motion of the patient; and the second response characteristic includes a low-frequency cutoff at a frequency greater than a frequency of the patient's respiration motion, but lower than a frequency of the patient's gross motion, such that the second accelerometer is configured to produce a second output signal in response to the gross motion of the patient, but not in response to the respiration motion of the patient.

In some variations, the first response characteristic includes a dynamic range with a noise floor sufficiently low such that the first accelerometer is configured to produce a first output signal in response to both a respiration motion of the patient and a gross motion of the patient; and the second response characteristic includes a low-frequency cutoff at a frequency greater than a frequency of the patient's respiration motion, but lower than a frequency of the patient's gross motion, such that the second accelerometer is configured to produce a second output signal in response to the gross motion of the patient, but not in response to the respiration motion of the patient.

In other variations, the first response characteristic includes a low-frequency cutoff at a frequency sufficiently low such that the first accelerometer is configured to produce a first output signal in response to both a respiration motion of the patient and a gross motion of the patient; and the second response characteristic includes a dynamic range with a low-end that is above the amplitude of the respiration motion of the patient but below the amplitude of a gross motion of the patient, such that the second accelerometer is configured to produce a second output signal in response to the gross motion of the patient, but not in response to the respiration motion of the patient.

Advantageously, the step of providing a first accelerometer includes providing a providing a first accelerometer having a first axis of sensitivity and a third axis of sensitivity, the first accelerometer configured to produce a first contributory signal in response to motion sensed by the first accelerometer along the first axis of sensitivity, and configured to provide a third contributory signal in response to motion sensed by the first accelerometer along the third axis of sensitivity; and the step of providing a second accelerometer includes providing a providing a second accelerometer having a second axis of sensitivity and a fourth axis of sensitivity, the second accelerometer configured to produce a second contributory signal in response to motion sensed by the second accelerometer along the second axis of sensitivity, and configured to provide a fourth contributory signal in response to motion sensed by the second accelerometer along the fourth axis of sensitivity; and the method further includes summing the first contributory signal and the third contributory signal to produce the first motion signal, and summing the second contributory signal and the fourth contributory signal to produce the second motion signal.

Some embodiments also include determining respiration rate of the subject by determining the number of cycles of the cyclical respiration signal over a predefined period of time. For example, the predefined period of time may be one minute, such that the respiration of the patient may be expressed in term of cycles per minute.

A system for detecting the respiration of a patient, includes a first accelerometer configured to be affixed to the patient so as to be subject to a gross motion of the patent and a respiration motion of the patient, the first accelerometer having a first response characteristic configured to produce a first output signal in response to both the respiration motion of the patient and the gross motion of the patient; a second accelerometer configured to be affixed to the patient so as to be subject to at least the gross motion of the patent, the second accelerometer having a second response characteristic configured to produce a second output signal in response to the gross motion of the patient, but not in response to the respiration motion of the patient; and a signal processing circuit configured to determine the difference between the second motion signal and the first motion signal and to produce a cyclical respiration signal, the respiration signal produced in response to the subject's respiration.

In some embodiments, the second accelerometer is disposed proximate to the first accelerometer such that the first accelerometer and the second accelerometer are subject to substantially identical respiration motion. For example, in some embodiments, the second accelerometer is disposed within 3 centimeters of the first accelerometer. In some embodiments, the first accelerometer and the second accelerometer are in a stacked configuration.

In contrast, in some embodiments the second accelerometer is disposed such that the second accelerometer and the first accelerometer are not subject to substantially identical respiratory motion. In some embodiments, the first accelerometer has a first axis of sensitivity and a third axis of sensitivity, the first accelerometer configured to produce a first contributory signal in response to motion sensed by the first accelerometer along the first axis of sensitivity, and configured to provide a third contributory signal in response to motion sensed by the first accelerometer along the third axis of sensitivity; the second accelerometer has a second axis of sensitivity and a fourth axis of sensitivity, the second accelerometer configured to produce a second contributory signal in response to motion sensed by the second accelerometer along the second axis of sensitivity, and configured to provide a fourth contributory signal in response to motion sensed by the second accelerometer along the fourth axis of sensitivity. In such embodiments, a first summing circuit is configured to receive and sum the first contributory signal and the third contributory signal to produce the first motion signal; and a second summing circuit is configured to receive and sum second contributory signal and the fourth contributory signal to produce the second motion signal.

## Brief Description of the Drawings

The foregoing features of embodiments will be more readily understood by reference to the following detailed description, taken with reference to the accompanying drawings, in which:

Fig. 1A and Fig. 1B schematically illustrate a human spine;

Figs. 2A-2C schematically illustrate features of embodiments of accelerometers as known in the art;

Fig. 2D schematically illustrates vectors of an axis of sensitivity of an accelerometer;

Fig. 2E schematically illustrates an accelerometer having several output terminals;

Figs. 3A-3C schematically illustrate a human at rest and at various stages of respiration;

Figs. 3D schematically illustrates an embodiment of a system for detecting respiration of a patient;

Fig. 4 schematically illustrates an embodiment of a respiration belt;

Fig. 5A schematically illustrates an embodiment of a signal processing system;

Figs. 5B-5F schematically illustrate embodiments of signal processing circuits;

Figs. 6A - 6C schematically illustrate an alternate embodiment of a respiration detection system;

Fig. 7A schematically illustrates the response of an accelerometer to respiration motion;

Figs. 7B and 7C schematically illustrate examples of the response of an accelerometer to both respiration motion and gross motion;

Fig. 7D schematically illustrates examples of the frequency characteristics of a patient's respiratory motion and the frequency characteristics of the patient's gross motion;

Figs. 7E and 7F schematically illustrate examples of the frequency response characteristics of two accelerometers;

Figs. 8A and 8B schematically illustrate examples of the response of another accelerometer to both respiration motion and gross motion;

Figs. 9A - 9C schematically illustrate methods of detecting respiration in a patient;

Fig. 10 schematically illustrates breathing data from a multiple-axis accelerometer;

Fig. 11A, Fig. 11B and Fig. 11C schematically illustrate alternate embodiments of accelerometer respiration sensing systems;

Fig. 12 schematically illustrates an alternate embodiment of a respiration sensing system;

Figs. 13A and 13B schematically illustrate an alternate embodiment of a respiration sensing system.

## Detailed Description of Specific Embodiments

Various embodiments provide a simple and direct way of measuring respiration in a patient by using two accelerometers on an unobtrusive abdominal band worn by the patient. The band holds one of the accelerometers near the patient's spine (herein, the "posterior" accelerometer), and holds the other accelerometer on the other side of the patient's body near the anterior side of the patient's abdomen (herein, the "anterior" accelerometer). Each of the accelerometers detects motions of the patient's body, but the anterior accelerometer more directly senses motions of the abdomen due to the patient's respiration (i.e., breathing). To the extent that either of the accelerometers detects the pull of gravity, both accelerometers will sense approximately the same gravitational influence. As such, motion attributable to the patient's respiration can be distinguished from other forces (e.g., the patient's motion, forces of gravity) by simply subtracting the motion sensed by the posterior accelerometer from the motion sensed by the anterior accelerometer.

In some embodiments, the two accelerometers may be identical. In other embodiments, however, the two accelerometers may be non-identical. For example, in some embodiments, the two accelerometers may have distinct response characteristics, which may make it easier to perform the signal processing.

Definitions. As used in this description and the accompanying claims, the following terms shall have the meanings indicated, unless the context otherwise requires:

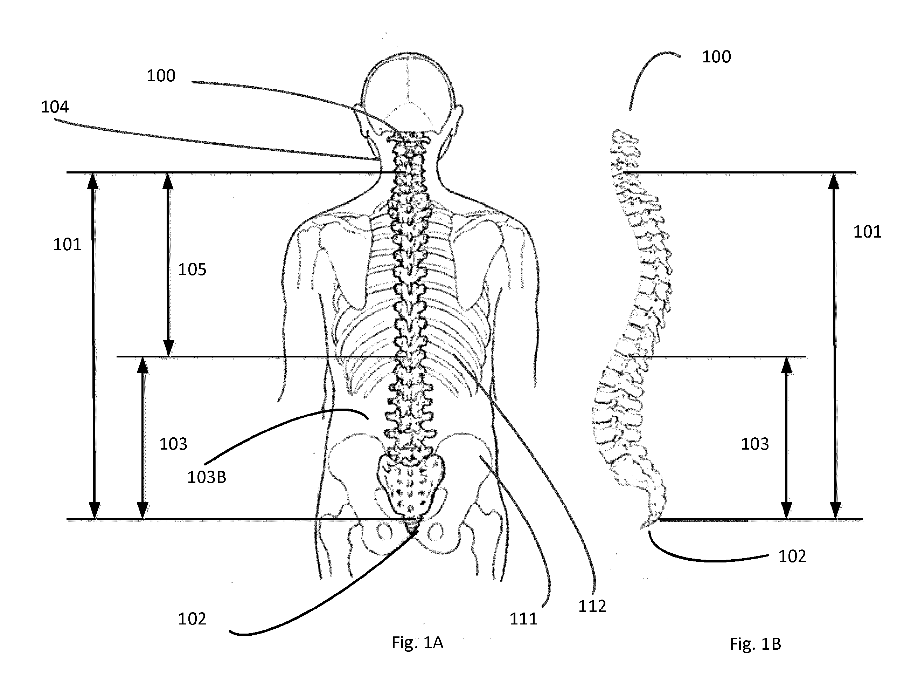

A "spinal region" means the portion of a human spine including the coccyx, and extending through the human's neck. A spinal region 101 of a spine 100 of a human patient is schematically illustrated in Fig. 1A and Fig. 1B.

A "lower spinal region" means the portion of a human spine 100 including the coccyx 102, and extending through the human's abdomen to approximately the location of the human's diaphragm as schematically illustrated as region 103 in Fig. 1A and Fig. 1B.

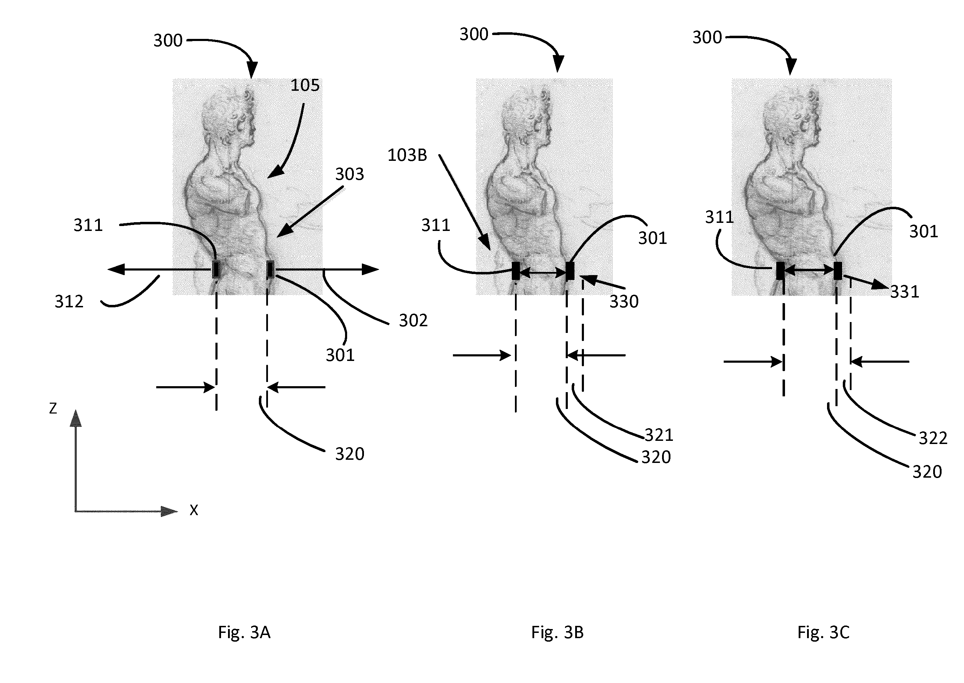

A "thorax" or "chest region" 105 means the portion of a human torso between the person's diaphragm 112 and the person's neck 104, as schematically illustrated in Fig. 3A. The location of the thorax 105 is schematically illustrated in Fig. 1A and in Fig. 3A.

A "lower abdominal region" (e.g., 303) is a portion of a human's abdominal region between the person's diaphragm and pelvis 111. The location 112 of a human's diaphragm is schematically illustrated in Fig. 1A. A lower abdominal region has an anterior side (for example, a person's belly button is located on the anterior side of the person's lower abdominal region) and a posterior side (which may be generally referred-to as the person's lower back 103B).

The term "affixed" when used in reference to a MEMS device and a portion of a patient's body means that the MEMS device is disposed such that movement of the portion of the patient's body is transmitted to, and able to be sensed by, the MEMS device. For example, in an extreme case, an accelerometer may be attached to a bone in the patient's body by securing the accelerometer with a surgical screw. As such, the accelerometer would be attached to the patient's body such that the accelerometer would be absolutely immovable with respect to the bone, and therefore motion of the bone could be easily and faithfully sensed by the accelerometer. However, such absolute attachment may not be necessary in all embodiments. For example, in some embodiments, one or more accelerometers 301, 311 may be attached to a an item of clothing, such as a shirt or belt, worn by the patient, such that the accelerometers are substantially immobile with respect to the patient's body. The amount of allowable motion of an accelerometer with respect to a patient's body may be determined by the needs of the system in which the accelerometer is employed, and thus may vary from one system to another, at the discretion of each system's designer. If an accelerometer is physically coupled to a patient's body such that the motion of the accelerometer with respect to the patient's body is acceptable to the system designer, then the accelerometer may be said to be affixed to the patient's body.

A "respiratory motion" is a motion of a part of a patient's body due to the patient's respiration. For example, respiratory motion may be motion of one part of the patient's body relative to another part of the patient's body, such as the motion of a point on the surface of a patient's abdomen with respect to a point on the patient's lower spine (i.e., in the lower spinal region), which motion occurs as the patient inhales and exhales. In addition, respiratory motion may be motion of one part of the patient's body between two positions, such as the motion of the patient's chest between a point when the patient has inhaled, and a point when the patient has exhaled.

A "gross motion" of a patient includes bodily motions other than a respiratory motion. For example, a subject's gross motion may be motion sensed by a MEMS sensor in response to the subject walking, running, or rolling over in bed.

The "sensitivity" of a MEMS device is the ratio of the device's output to a given stimulus. For example, an accelerometer may produce a voltage output in response to an acceleration applied to the accelerometer. As such, the sensitivity of the accelerometer could be expressed in volts per g (volts/g) or millivolts per g (mV/g), where "g" denotes the quantity of acceleration due to gravity on the surface of the Earth (i.e., 9.8 meters/second-squared). For example, if a first accelerometer produces an output of 2 volts in response to a $1g$ acceleration, then the first accelerometer could be described as having a sensitivity of 2V/$g$. Similarly, if a second accelerometer produces an output of 3 volts in response to a $1g$ acceleration, then the second accelerometer could be described as having a sensitivity of 3V/g.

The "dynamic range" of a MEMS device is the difference between the largest stimulus that the device can accurately transduce, and the smallest stimulus that the device can accurately transduce. The largest stimulus that the device can accurately transduce may be defined as the signal amplitude at which the output of the MEMS device become non-linear or otherwise beyond the ability of the MEMS device to produce an output signal that accurately reports the characteristics of the stimulus, and may be limited, for example, by the stiffness of the suspension system of the movable element within the MEMS device, or the extent to which a movable element within the MEMS device can move. The smallest stimulus that the device can accurately transduce may be limited, for example, by the device's noise floor, if the output signal from the device in response to a given stimulus is lower than the device's noise floor. The noise floor of a given MEMS device is typically specified by the device's manufacturer.

The "bandwidth" of a MEMS device is the difference between the highest frequency motion the device can accurately transduce, and the lowest frequency motion that the device can accurately transduce. The frequencies that a device can accurately transduce may be limited, for example, by the mass of the device's beam, or the stiffness of the suspension of the device's beam. A frequency may be below the bottom-end of the device's bandwidth (i.e., the device's low-frequency cutoff) if the device's response to a motion of that frequency is half (-3db) or less than the device's response to a higher frequency. The bandwidth of a given MEMS device is typically specified by the device's manufacturer.

A "response characteristic" of a MEMS device means the characteristics of a response of the MEMS device to an input stimulus. A response characteristic may also be described as the transfer function of the MEMS device. The response characteristic of a MEMS device may be influenced by the sensitivity, dynamic range, bandwidth or low-frequency cutoff, and noise floor of the MEMS device. For example, two accelerometers that have distinct dynamic ranges will have distinct response characteristics to an acceleration that is within the dynamic range of one of the accelerometers, but not within the dynamic range of the second of the accelerometers.

The term "patient" refers generally to a breathing organism, and is not limited to humans, or even to humans seeking medical attention. For example, a patient may be a non-human animal.

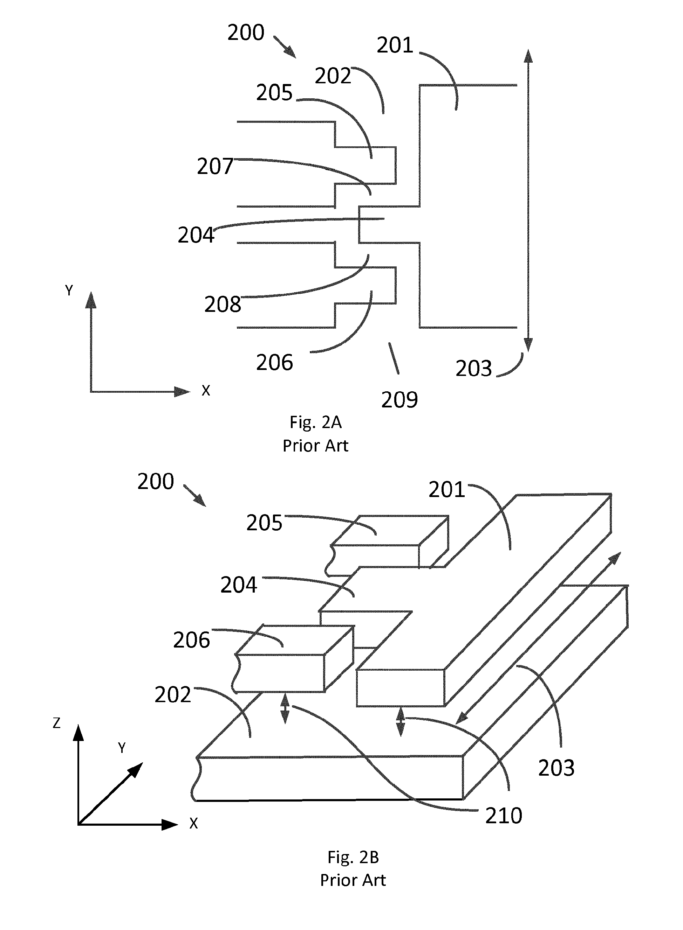

Fig. 2A and fig. 2B schematically illustrate an example of a MEMS accelerometer 200, although the embodiments described herein are not necessarily limited to the use of accelerometers, and are not limited to the use of the accelerometer described in connection with Figs 2A and 2B. Figure 2A schematically illustrates a single cell of a prior art differential capacitor 209 that could be used in an accelerometer. The accelerometer 200 has a beam 201 suspended above, and substantially parallel to, a substrate 202. The beam 201 is movable along axis 203, which may be known as the "axis of sensitivity" of accelerometer 200, because this is the axis for which the accelerometer 200 senses acceleration and for which the accelerometer produces an electrical output signal. In other words, an accelerometer (e.g., 200) senses motion along its axis of sensitivity, and produces an output signal in response to such motion.

Some accelerometers have more than one axis of sensitivity. For example, some accelerometers have two or three axes of sensitivity. Indeed, in some multiple-axis accelerometers, the axes are mutually orthogonal (or substantially mutually orthogonal) to one another.

In addition, an axis of sensitivity (e.g., 203) may be characterized as including a positive direction of sensitivity, such that the accelerometer produces a positive output in response to an acceleration in the same direction as the positive direction of sensitivity, and a negative direction of sensitivity along the same line as the positive direction of sensitivity, such that the accelerometer produces an output that is the inverse of the positive output in response to an acceleration in the opposite direction as the positive direction of sensitivity. In other words, such an accelerometer might produce an output of 2V/g for an acceleration in the direction of the positive direction of sensitivity, and an output of -2V/g for an acceleration of equal magnitude in the direction of the negative direction of sensitivity. In a system with two accelerometers, the two accelerometers could be disposed such that their respective positive directions of sensitivity are diametrically opposed, so the two accelerometers will produce output signals of opposite polarity in response to the same applied acceleration (in other words, the output signals would be complementary with respect to one another). As such, the difference between the respective output signals could be produced by simply summing the two output signals.

The beam 201 includes at least one finger 204 that moves with the beam. Movable finger 204 is an electrode, and a plurality of such movable fingers may be connected to one another to form a larger movable electrode. Adjacent to the beam are fixed fingers 205 and 206, which are also electrodes, and which are electrically independent in illustrative embodiments. The fixed fingers 205 and 206 are meshed or inter-digitated with the movable finger 204 to form a differential capacitor 209 comprising a first capacitor 207 formed from the movable finger 204 and fixed finger 205, and a second capacitor 208 formed from the movable finger 204 and fixed finger 206.

When a force is applied to the substrate 202 along axis 203, the substrate 202 and fixed fingers 205 and 206 move in the direction of the applied force, while the beam 201 inertially, at first, remains in its prior position. When the force is in one direction, the separation between movable finger 204 and fixed finger 205 increases, decreasing the capacitance in the first capacitor 207. Conversely, the separation between movable finger 204 and fixed finger 206 decreases, increasing the capacitance in the second capacitor 208. When the force on the substrate 202 is in the opposite direction, the effect on the first and second capacitors 207 and 208 is reversed.

It should be noted that various embodiments apply to MEMS devices with different variable capacitors. For example, illustrations may apply to variable capacitors having one fixed plate or finger and one movable plate or finger. Any discussion of a system with two fixed fingers is illustrative and not intended to be limiting.

A perspective view of some portions of a differential capacitor cell of Figure 2A is shown in Figure 2B. Not all structures are shown. Double-headed arrows 210 schematically illustrate the suspension of the beam 201, and the height of fixed fingers 205 and 206, above substrate 202.

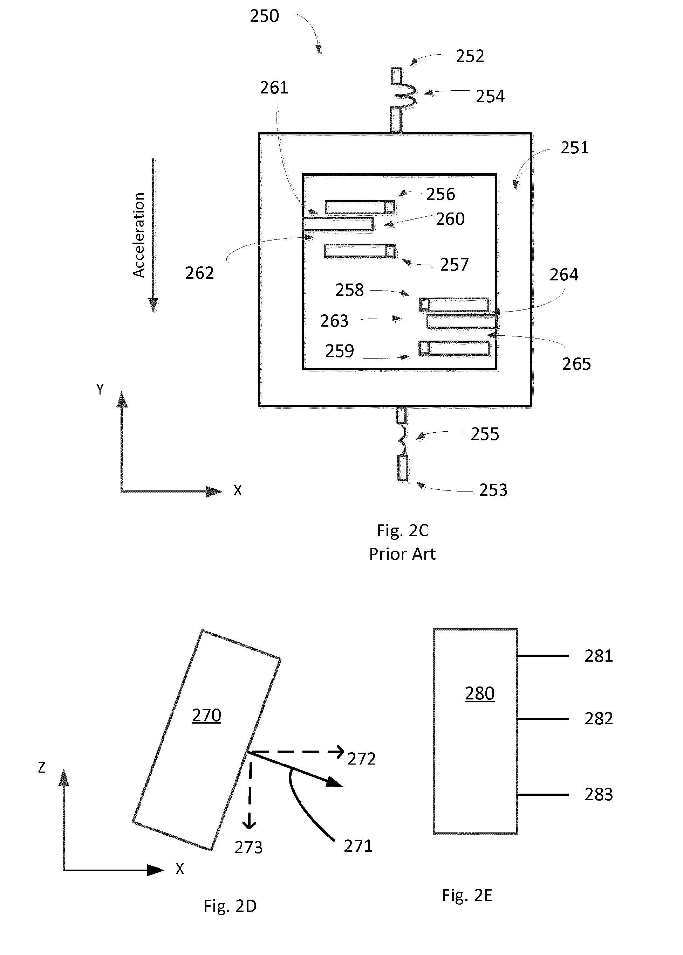

An alternate arrangement 250 of the beam 251, and illustrative movable fingers 260 and 263 and fixed fingers 256, 257, 258 and 259, is schematically illustrated in Figure 2C. The beam 251 is rectangular, and suspended above a substrate 266 (not shown) from the anchors 252 and 253 by the springs 254 and 255, respectively. The beam 251 is movable in the Y axis, and the axis of sensitivity of the accelerometer 250 is parallel to the Y axis.

The movable fingers 260 and 263 extend from sides of the internal wall of the beam. The stationary fingers 256, 257, 258 and 259 are fixed to the substrate 266 and are in the plane of the beam 251. The fixed fingers 256 and 257, along with movable finger 260, form first capacitor 261 and second capacitor 262, respectively. Similarly, fixed fingers 258 and 259, and movable finger 263, form capacitors 264 and 265, respectively. In Fig. 2C, the accelerometer is schematically illustrated as subject to an acceleration in the -Y direction, such that the beam is nearer to the anchor 252 than to anchor 253, and spring 254 is compresses and spring 255 is extended.

Fig. 2D schematically illustrates an accelerometer 270 having an axis of sensitivity 271. If the accelerometer is subject to acceleration along an the X axis, which is not parallel to the axis of sensitivity 271, the accelerometer 270 will still produce an output signal representing a portion of the magnitude of the applied acceleration. As such, it may be said that the axis of sensitivity 271 of an accelerometer (e.g., 270) may have a vector of sensitivity (e.g., 272) parallel to the X axis (and also a vector of sensitivity 273 perpendicular to the X axis).

Fig. 2E schematically illustrates a three-axis accelerometer 280 that has three output terminals (281, 282, 283), each of which is configured to produce an output signal (e.g., a voltage signal or a current signal or a digital signal) in response to a measured acceleration.

For example, output terminal 281 may produce a signal in response to acceleration along an X-axis, output terminal 282 may produce a signal in response to acceleration along a Y-axis, and output terminal 283 may produce a signal in response to acceleration along a Z-axis (see, e.g., Fig. 2B). Accelerometers with fewer than three axes of sensitivity may have fewer output terminals. For example, a single-axis accelerometer would have only a single output terminal 281, and a dual-axis accelerometer would have two output terminals 281 and 282. As such, accelerometer 280 may represent any of the accelerometers described in any of the embodiments herein.

As described above, an accelerometer is a type of transducer that converts a motion, such as a motion of a human subject, or a part of a human subject, into an electrical signal. Other types of MEMS devices, such as gyroscopes for example, are also transducers that convert a motion, such as a motion of a human subject, or a part of a human subject, into an electrical signal. In practice, transducers have a dynamic range defined by a "top end" and a "bottom end."

The bottom-end of a transducer's dynamic range is determined primarily by electrical noise signals inherent in the transducer. This electrical noise may be known as "Brownian" noise. The electrical signal output by the transducer includes a component representing the incident motion signal and a component representing the noise. If the amplitude of the noise signal approaches that of the motion signal, the motion signal may not be distinguishable from, or detectable from within, the noise. In other words, the noise may overwhelm the signal. The point where the noise signal overwhelms the motion signal is known as the noise floor, and the bottom-end of the dynamic range is a function of the noise floor of the transducer.

The top-end of a transducer's dynamic range is determined by the distortion present in the output electrical signal. In an ideal accelerometer, for example, the accelerometer's output will always be an undistorted representation of the incident acceleration forces acting on the accelerometer. In real accelerometers, however, as the incident acceleration grows more powerful, the electrical signal output from the accelerometer begins to distort because the accelerometer's movable member (or "beam") cannot perfectly respond to high acceleration levels, and the output becomes nonlinear. At some point, the level of distortion exceeds the system tolerance, so accelerations above that point fall outside the dynamic range of the accelerometer. The point of unacceptable distortion must be determined by the system designer as a function of the system being designed. Some applications may tolerate higher distortion than others.

Thus, a transducer's dynamic range is determined primarily by the noise floor at the bottom-end, and the point of unacceptable distortion at the top-end.

Fig. 3A schematically illustrates a human patient 300 not engaging in respiration. Fig. 3B schematically illustrates a patient 300 exhaling, and Fig. 3C schematically illustrates a patient 300 inhaling. In each of Figs. 3A-3C, two accelerometers 301, 311 are affixed to the patient 300. The accelerometers 301, 311 may of the type described above, for example. More specifically, an anterior accelerometer 301 is affixed to the abdominal region 303 of the patient 300, while a posterior accelerometer 311 is affixed to the lower spinal region 103 of the patient 300, such that the patient's abdomen 303 is disposed between the anterior accelerometer 301 and the posterior accelerometer 311. In some embodiments, the axis of sensitivity 312 of the posterior accelerometer 311 is perpendicular to the patient's back, and in some embodiments the axis of sensitivity 302 of the anterior accelerometer 301 is parallel to the axis of sensitivity 312 of the posterior accelerometer 311.

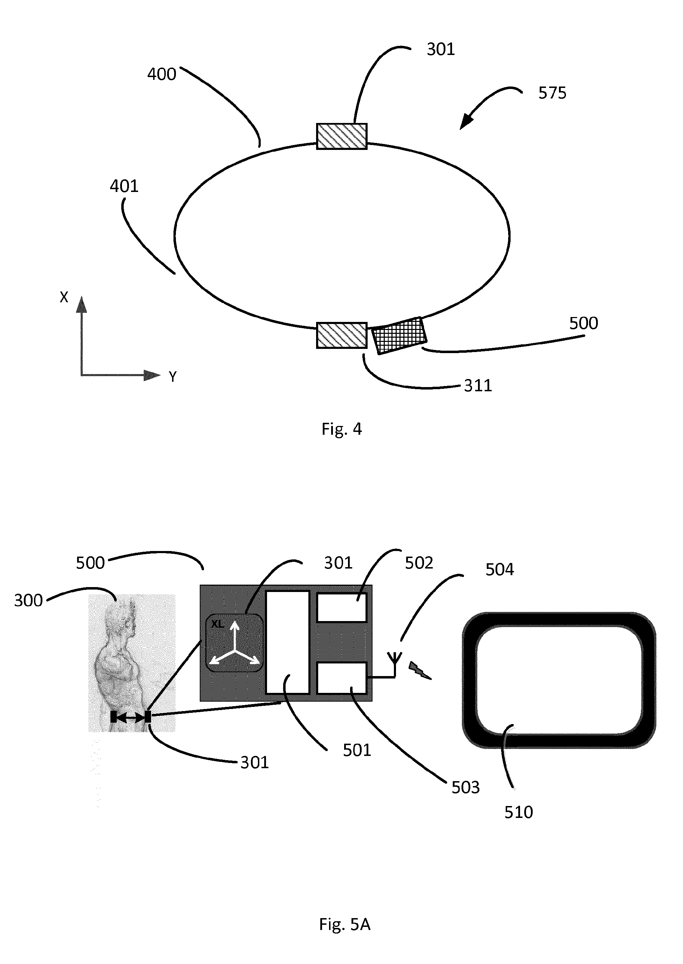

In some embodiments, the accelerometers 301 and 311 may be affixed to the patient 300 by a sensor belt or band 400 as schematically illustrated in Fig. 4. The sensor includes a belt strap 401 configured to fit around, and be secured to, the patient 300. For example the band 400 may be secured around the patient's abdomen 303, so as to affix the two accelerometers 301 and 311 into the positions described above.

The band 400 may include other elements of a respiratory measurement system 575. For example, in addition to accelerometers 301, 311, a system 575 may include elements such as a power source 502 and/or signal processing circuitry. The power source 502 may include one or more batteries or other power sources. Some embodiments of signal processing circuitry 500 may include, for example, one or more buffers or amplifiers 521, signal filters 522, analog-to-digital converters 523, microprocessor or digital signal processor integrated circuits 501 (each of which may be referred to as a "DSP"), wireless transmitter circuits 503, phase shifters 527, or other circuitry to receive and process the outputs of the accelerometers 301 and 311. In some embodiments, a digital signal processor integrated circuit 501 may control one or more analog-to-digital converters 523, for example via one or more control signals 529, so that the digital signal processor integrated circuit 501 can control and record the time at which a signal was sampled.

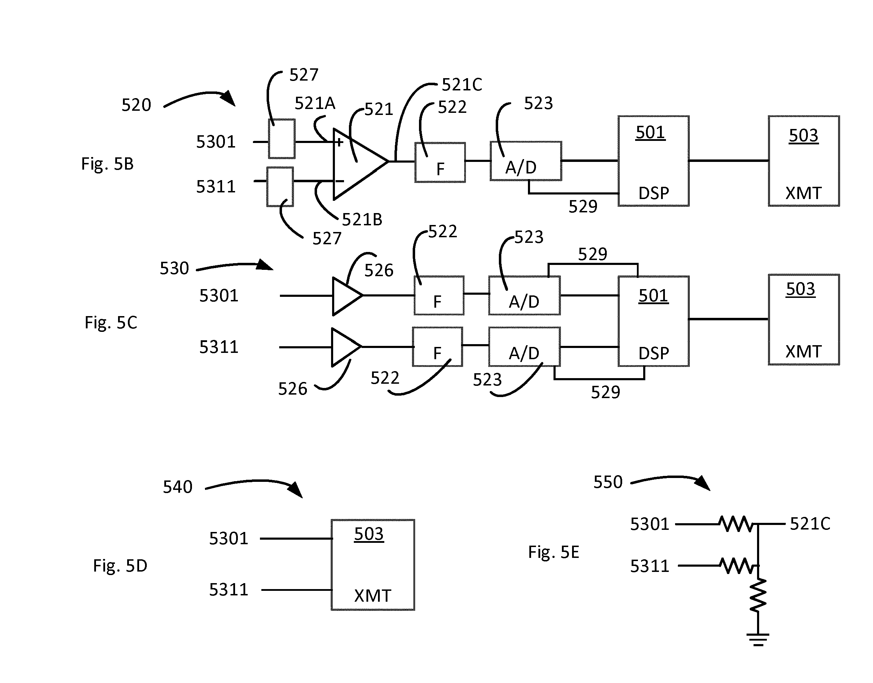

For example, as schematically illustrated in Fig. 5B, the circuitry 520 may include a differential amplifier 521 configured to receive, at its differential inputs 521A and 521B, the outputs 5301 and 5311 of the accelerometers 301 and 311, respectively, and configured to produce an output signal at its output terminal 521C, which output signal has an amplitude defined by the difference between the outputs 5301 and 5311 of the accelerometers 301 and 311. In some embodiments, if the output signals 5301 and 5311 from accelerometers 301, 311 are out of phase (e.g., the output of one of the accelerometers 301, 311 is delayed relative to the output of the other one of the accelerometers 301, 311), the circuit 520 may include one or more delay elements to delay one or both of the signals 5301, 5311, so as to lessen the difference in the times that a response to a motion sensed by both accelerometers 301, 311 arrives at the amplifier 521, for example.

The circuitry 520 also includes an analog-to-digital converter 523 configured to convert the output signal from the amplifier 521 to produce a digital signal, and provide that digitized signal to computer processor hardware 501, such as a digital signal processor integrated circuit (i.e., a "DSP") programmed to filter the outputs 5301 and 5311 of the accelerometers 301 and 311. If the two accelerometers 301 and 311 do not have similar sensitivities, their outputs may be scaled by amplifier 521 in circuit 520, or by either of optional amplifiers 526 in circuit 530 of Fig. 5C, for example such that the outputs 5301 and 5311 of the accelerometers 301 and 311 have similar or identical amplitudes. Alternately, or in addition, one or both of the amplifiers 526 may introduce a delay or phase shift into the outputs 5301 and 5311 so as to mitigate any delay or phase difference between them, as described previously.

In some embodiments, the outputs 5301 and 5311 of the accelerometers 301 and 311 may be summed, for example by a summing junction 550 as schematically illustrated in Fig. 5E. For example, the summing junction 550 may be substituted for amplifier 521 in circuit 520 of Fig. 5B. The design, construction and use of a summer junction, such as the three-resistor summing junction 550, are well known in the art and are therefore not described further here.

Some embodiment include one or more optional analog filters 522 to filter one or both of the outputs 5301 and 5311 of the accelerometers 301 and 311 (Fig. 5C), or to filter the output of the amplifier 521. For example, such filters may be high-pass filters configured to reduce the amplitude of signal components that represent low-frequency respiration motion. Some embodiments omit the amplifier 521, as schematically illustrated by circuit 530 in Fig. 5C.

In addition, some embodiments may include a transmitter circuit 503 configured to receive an output from the processor 501 and to wirelessly transmit signals to an external receiver 510 (e.g., Fig. 5A), such as a laptop computer, mobile phone, tablet computer, or computer server, as schematically illustrated in Fig. 5B and Fig. 5C. Such signals may include the digitized outputs 5301 and 5311 of the accelerometers 301, 311, or a digital output produced from the outputs 5301 and 5311 of the accelerometers 301, 311 by a signal processor 501. Alternately, some embodiments may transmit outputs 5301 and 5311 of the accelerometers 301, 311 in the analog domain, for example as schematically illustrated by circuit 540 in Fig. 5D.

Returning to Fig. 3A, as schematically illustrated, the posterior accelerometer 311 and the anterior accelerometer 301 are disposed such that the patient's abdomen 303 is between them, or in other words, such that the axes of sensitivity 302 and 312 both pass through the patient's abdomen. As such, there is a nominal distance 320 between them.

In operation, as the patient 300 breathes, the posterior accelerometer 311 remains substantially immobile with respect to the patient. As such, the posterior accelerometer 311 senses the gross motion of the patient 300, but does not sense the respiratory motion of the patient.

In contrast, the anterior accelerometer 301 moves in response to the patient's respiration. More specifically, the anterior accelerometer 301 moves closer to the posterior accelerometer 311 when the patient exhales, as schematically illustrated by arrow 330 in Fig. 3B. As shown in Fig. 3B, the distance 320 between the accelerometers 301 and 311 has closed, or been reduced, by an amount 321 as a result of the patient 300 having exhaled.

Similarly, the anterior accelerometer 301 moves away from the posterior accelerometer 311 when the patient inhales, as schematically illustrated by arrow 331 in Fig. 3C. As shown in Fig. 3C, the distance 320 between the accelerometers 301 and 311 has widened, or been increased, by an amount 322 as a result of the patient 300 having inhaled.

As the patient 300 breathes, the distance 320 between the accelerometers 301, 311 increases and decreases cyclically, in sympathy with the patient's respiration. As a practical matter, not all human abdomens are as flat as the abdomen 303 of patient 300. In such circumstances, the axis of sensitivity 302 of the anterior accelerometer 301 may not be exactly parallel to the axis of sensitivity 312 of the posterior accelerometer 311. However, the signals output from those accelerometers 301, 311 will still be useable in determining the respiration rate of the patient 300 as long as the axis of sensitivity 312 of the anterior accelerometer 302 has a vector of sensitivity parallel to the axis of sensitivity 312 of the posterior accelerometer 311.

The patient's respiration rate may be determined by, among other ways, by counting the number of peaks in the patient's respiration signal over a given time interval. For example, see the peaks 701-1, 701-2 ... 701-12 in the signal 701 ofFig. 7A.

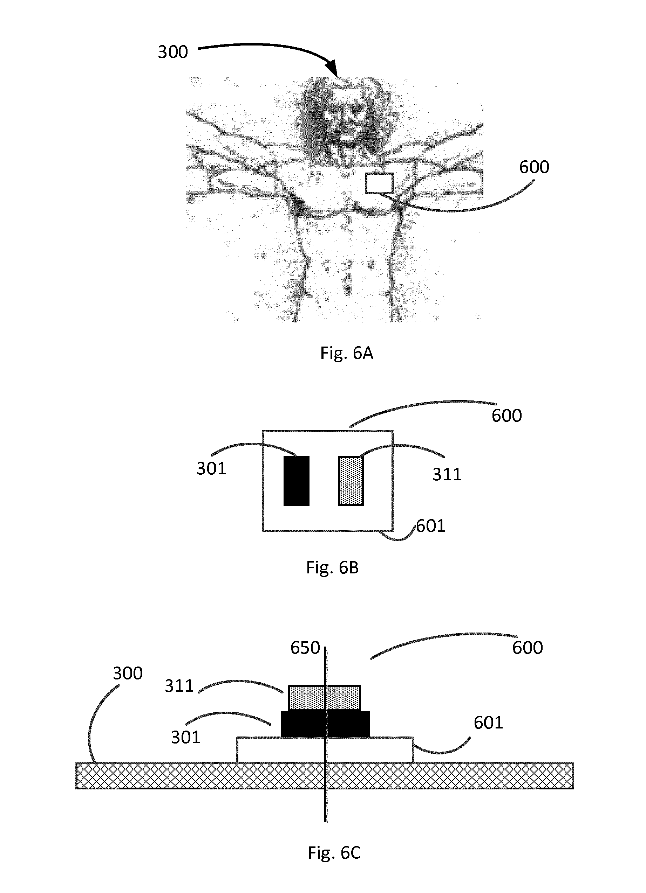

Some embodiments detect respiration without needing to sense motions at different locations on a patient, and/or without having to be disposed at different places on a patient's body. For example, an alternate embodiment 600 includes two accelerometers 301, 311 with different (i.e., non-identical) response characteristics, as schematically illustrated in Fig. 6A and 6B. Both accelerometers 301 and 311 are affixed to the subject 300, and both therefore are subjected to one or more motions of the subject (e.g., gross motion and/or respiratory motion). In some embodiments, the accelerometers 301 and 311 may be positioned very close to one another, since the operation of the system 600 does not depend on sensing motions from two distinct positions on the body of the patient 300. In other words, some embodiments may detect a respiration signal by sensing a motion (a "compound motion") that has a first component generated by gross motion and a second component generated by respiratory motion.

To that end, in some embodiments the accelerometers 301 and 311 may be positioned within 1 centimeter, or 2 centimeters or 3 centimeters of one another. In some embodiments, the accelerometers 301 and 311 may even be in a stacked configuration (e.g., a line 650 normal to the patient's body 300 would pass through both of the accelerometers 301 and 311, as schematically illustrated in Fig. 6C). Indeed, in some embodiments, both accelerometers 301 and 311 are mounted on the same substrate 601, and may even be fabricated on the same silicon die or substrate 601.

However, because the two accelerometers have non-identical response characteristics, the outputs of the respective accelerometers will not be identical, even if they are both subjected to the same motions. In preferred embodiments, one of the accelerometers produces an output signal in response to both gross motion and respiration motion, while the other accelerometer produces an output signal in response only to the gross motion. As such, the outputs of the two accelerometers may be compared or processed to determine the contribution made only by the respiration motion.

For example, in one embodiment, the two accelerometers 301, 311 may have different (i.e., distinct; non-identical) dynamic ranges, such that a patient's respiration motion is within the dynamic range of one of the accelerometers (and therefore is represented in the output signal from that accelerometer), but not within the dynamic range of the second accelerometer (and therefore is not represented in the output signal from that accelerometer). For purposes of this description and the accompanying claims, if a stimulus is outside of the dynamic range of a transducer, then that transducer is deemed to have a response characteristic that is not configured to transduce (e.g., produce a response to) that stimulus.

In an alternate embodiment, the two accelerometers may have different frequency ranges , such that the low-frequency (e.g., < 1Hz; < 5Hz; < 10Hz) of a respiration motion is within the bandwidth of that accelerometer (and therefore is represented in the output of that accelerometer), but is not within the bandwidth of the other accelerometer (and therefore is not represented in the output signal from that accelerometer). For purposes of this description and the accompanying claims, if a stimulus is below the low frequency cutoff of a transducer's bandwidth, then that transducer is deemed to have a response characteristic that is not configured to transduce (e.g., produce a response to) that stimulus.

In yet other embodiments, the two accelerometers have distinct dynamic ranges (e.g., noise floors) and also have distinct bandwidths, such that respiration motion is within the response characteristic of that accelerometer (and therefore is represented in the output of that accelerometer), but is not within the response characteristic of the other accelerometer (and therefore is not represented in the output signal from that accelerometer).

In yet other embodiments, a first accelerometer (e.g., 301) may have a dynamic range such that the first accelerometer is configured to transduce both respiration and gross motion, and the second accelerometer (e.g., 311) may have a low-frequency cutoff that is above the frequency of the respiration motion such that the second accelerometer (311) is configured to transduce a gross motion but not a respiration motion.

Alternately, in another embodiment, a first accelerometer (e.g., 301) may have a frequency range such that the first accelerometer is configured to transduce both respiration and gross motion, and the second accelerometer (e.g., 311) may have a dynamic range such that the second accelerometer (311) is configured to transduce a gross motion but not a respiration motion.

In general, two transducers (e.g., two accelerometers) that have different response characteristics will produce output signals that are distinguishable from one another, even in response to the same stimulus. Embodiments of such systems 600 are schematically illustrated in Fig. 6A and Fig. 6B, and are described below.

In some embodiments, the two accelerometers 301, 311 may both be subject to the same motions or substantially the same motions. Two motions may be considered to be substantially the same (or signals representing the two motions are substantially the same) if their waveforms are identical, and/or if their respective energies are within 90 percent or 95 percent of each other, although higher or lower ratios of their energies (i.e., greater than 95 percent or less than 90 percent) may be acceptable in some applications, as determined by the engineer designing the system or the person operating the system. The ratio of the two energies may be higher or lower in various embodiments, for example according to the requirements and tolerances of the system being designed. A person of ordinary skill in the art will, after having read this disclosure, be able to determine an acceptable ratio of the two energies for determining whether two motions (or signals representing the two motions) are substantially the same.

Also, two stacked accelerometers, or two accelerometers closely mounted on a common substrate (e.g., 601) may be deemed to sense the same motion. For example, the two accelerometers may be affixed to the patient in close proximity to one another, and therefore may not need to be affixed to portions of the patient's body that move differently in response to respiration, or be affixed to the anterior and posterior portions of the patient's body or otherwise positioned such that a portion of the patient's body is physically between the two accelerometers. Generally, the various embodiments 600 do not require that the two accelerometers 301, 311 be subjected to different stimuli. Indeed, in some embodiments, the two accelerometers may be enclosed within a single package, and may even on the same substrate, such as silicon or silicon-on-insulator substrate for example.

Example 1 (different noise floors)

An example of an embodiment system 600 having two accelerometers with distinct response characteristics, and in particular, with distinct noise floors, is described below. Although an embodiment of a system 600 having two accelerometers with distinct noise floors produces similar signals, those signals are not identical and can be processed in the ways described below.

The nature of respiration is that it is cyclical, and therefore a signal representing respiration is also cyclical, although may not be precisely periodic. As such, a patient's respiration rate may be determined, for example, by determining the number of cycles of a respiration signal occurring in a given time interval. Also, typically, a person's respiration motions are small relative to the person's gross motions.

In system 600, the first accelerometer 301 has a noise floor that is below the amplitude of the respiration motion, so that the first accelerometer 301 produces an output signal having a component in response to a respiration motion.

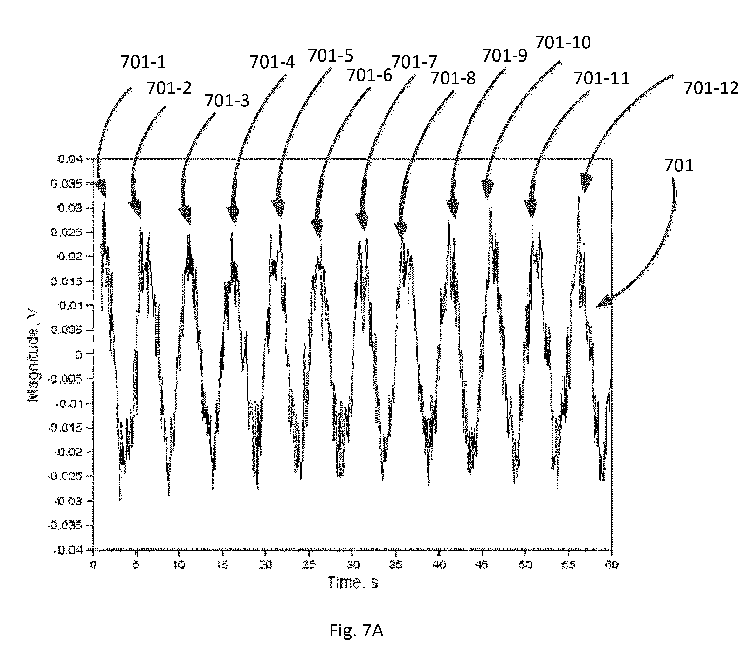

For example, as shown in Fig. 7A, an accelerometer 301 might produce a motion signal 701 with an amplitude of 0.025 volts/g (which equals 0.05 volts peak-to-peak) in response to a respiration signal. The signal 701 of Fig. 7A schematically illustrates 12 cycles over a period of sixty seconds, as evidenced for example by the twelve peaks (701-1, 701-2 ... 701-12). As such, the respiration rate of the respiration indicated by signal 701 is 12 cycles per minute (or 12/60 = 0.2 Hz).

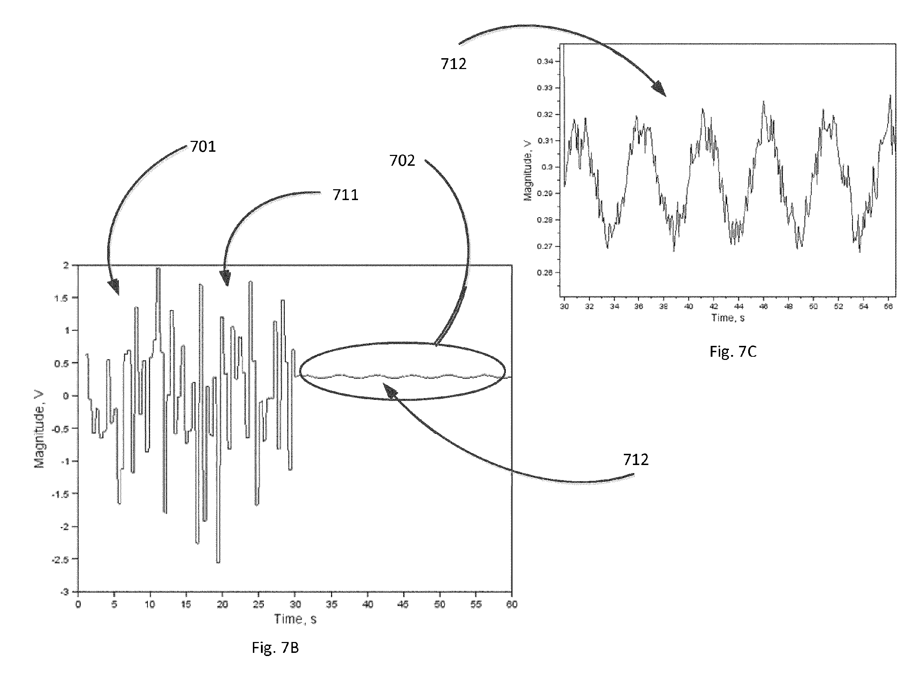

In practice, the first accelerometer 301 will also transduce the patient's gross motion, as schematically illustrated by signal segment 711 of the motion signal 701, in Fig. 7B. Such gross motion might produce a contribution to the motion signal 701, in addition to the respiration motion, such that the motion signal 701 has an amplitude of 2.25 volts (i.e., 4.5 volts peak-to-peak), as shown in Fig. 7B for example. The portion of the output of the first accelerometer 301 produced in response to such gross motion may be referred to as an "aggressor" signal, or simply as an "aggressor."

As such, the portion 712 of the motion signal 701 produced by the first accelerometer 301 in response to the respiration motion, as schematically illustrated in Fig. 7C, is significantly smaller than the portion of the motion signal 701 produced by the same accelerometer 301 in response to the patient's gross motion, even though both the respiration motion and the gross motion are within the response characteristic of the first accelerometer 301 (i.e., the accelerometer 301 is capable of sensing both the respiration motion and the gross motion; for example the motion signals are not below the noise floor of the accelerometer 301). As illustrated in Fig. 7B, the total motion signal 701 in segment 711 has an amplitude of approximately 2.25 volts (3.4 volts peak-to-peak), and as illustrated in Fig. 7C, the signal 712 has an amplitude of approximately 0.025 volts (0.32V - 0-.27V = 0.05 V peak-to-peak). As such, in the foregoing example, the ratio of the two (motion due to respiration and gross motion) is approximately 100:1 (2.25: 0.025).

In practice, the output of the first accelerometer 301 will also include some noise. The noise output of an accelerometer is typically expressed in units of micro-g per root Hertz, and is typically specified by the manufacturer of the accelerometer. In the present embodiment, both the first accelerometer 301 and the second accelerometer 311 have similar noise density. In preferred embodiments, the ratio of the noise density of the first accelerometer to the noise density of the second accelerometer is between 0.1 and 10. In other words, the specified noise density of one accelerometer should not be significantly more than (e.g., in some embodiments, not more than ten times) the specified noise density of the other accelerometer.

As such, the output of the first accelerometer 301 in response to respiration motion and gross motion is motion signal 701 schematically illustrated in Fig. 7B, and may be described by the following equation (1):

$$(1) \quad Vout1 = V\_respiration1 + V\_aggressor1 + V\_noise1$$

As schematically illustrated in Fig. 7B, all three of the foregoing components appear in the segment 711 of signal 701. However, in this example, the gross motion stops at the 30 second point, after which the segment 712 of the signal 701 includes only the response of accelerometer 301 to the respiration motion and noise, as schematically illustrated in Fig. 7C, which schematically illustrates an enlarged portion of signal 701 from oval window 702.

In system 600, the second accelerometer 311 has a response characteristic that is distinct from the response characteristic of the first accelerometer 301. In this embodiment, the response of the second accelerometer 311 to a respiration motion is below the noise floor of the second accelerometer 311.

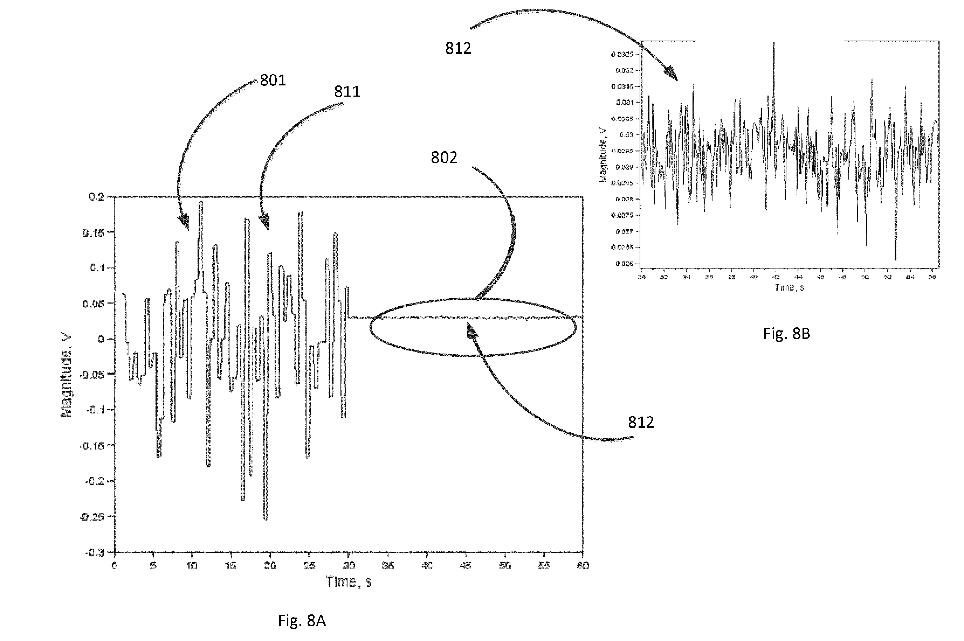

As such, the output 5311 of the second accelerometer 311 in response to respiration motion and gross motion is a signal 801 schematically illustrated in Fig. 8A, and may be described by the following equation (2):

$$(2) \ \text{Vout2} = \text{V\_aggressor2} + \text{V\_noise2}.$$

It should be noted that Vout2 does not include a response to respiration (i.e., there is no "V_respiration2"). This is because, for purpose of this example, the respiration signal is outside of the response characteristic (and specifically, below the noise floor of) the second accelerometer 311.

As schematically illustrated in Fig. 8A, both of the foregoing components appear in the segment 811 of signal 801. However, the gross motion stops at the 30 second point, after which the segment 812 of the signal 801 includes only noise output of the accelerometer 311, as schematically illustrated in Fig. 8B, which schematically illustrates an enlarged portion of signal 801 (i.e., segment 812) from oval window 802.

A signal 712 representing the respiration motion may be extracted from the signals output from the first accelerometer 301 and the second accelerometer 311. In some embodiments, the respiration signal may be determined by subtracting the output of the second accelerometer 311 from the output of the first accelerometer 301, as expressed by equation (3):

$$(3) \ \text{V\_respiration\_signal} = \text{Vout1} - \text{Vout2} =$$

$$\text{V\_respiration1} + \text{V\_aggressor1} + \text{V\_noise1} - \text{V\_aggressor2} - \text{V\_noise2}$$

If the aggressor signal is above the noise floor of both accelerometer 301 and accelerometer 311, and if both accelerometer 301 and accelerometer 311 produce output signals having the same volts/g, then V_aggressor1 will be equal to V_aggressor2. However, if accelerometer 301 and accelerometer 311 do not produce output signals having the same volts/g amplitude, then the output of one of the accelerometers 301, 311 could be amplified so that the amplitude of V_aggressor1 is equal to the amplitude of V_aggressor2. For example, such amplification could be performed by either or both of amplifiers 526, as schematically illustrated in Fig. 5C.

As such, the equation for the respiration signal reduces to equation (4):

$$(4) \ \text{V\_respiration\_signal} = \text{V\_respiration1} + \text{V\_noise1} - \text{V\_noise2}.$$

Thus, even though the respiration signal includes some noise, the respiration signal is sufficient to determine the respiration rate of the subject.

It should be reiterated that, in Example NF, the response of the second accelerometer 311 (Vout2) does not include a component in response to respiration (i.e., there is no "V_respiration2"). However, if the output signal (Vout2) of accelerometer 311 does include a component produced in response the respiration (i.e., "V_respiration2"), then the system might still produce an output useable for the purposes described herein, although the presence of that component might degrade the performance of the system. For example, such an output signal may be represented by equation 2':

$$(2') \ \text{Vout2} = \text{V\_respiration2} + \text{V\_aggressor2} + \text{V\_noise2}.$$

In such a case, the output of the system could be represented by equation (3'):

(3') V_respiration_signal = Vout1 – Vout2 =

V_respiration1 + V_aggressor1 + V_noise1 - V_respiration2 - V_aggressor2 -

V_noise2.

As shown by equation (3'), the respiration signal V_respiration2 produced by accelerometer 311 detracts from the respiration signal V_respiration1 produced by accelerometer 301. Although the system would still produce an output (V_respiration_signal), that output may not have the fidelity of the signal represented by equation (4). Indeed, in a worst case scenario, V_respiration1 = V_respiration2. In that case, the system output would not include a component representing respiration, as shown by equation (4'), in which the respiration components (V_respiration1; V_respiration2) have cancelled each other and the aggressor components (V_aggressor1; V_respiration2) have cancelled each other, leaving only noise:

(4') System Output = V_noise1 - V_noise2.

Therefore, in view of the foregoing disclosure, a system designer would know to select the accelerometers 301 and 311 such that accelerometer 301 produces an output signal (Vout1) having a component produced by respiration (V_respiration1), and such that accelerometer 311 produces an output (Vout2) that does not include a component produced by respiration (V_respiration2) equal to the corresponding component (V_respiration1) in the output of accelerometer 301, and preferably produces an output (Vout2) that does not include any component produced by respiration (i.e., V_respiration2 = 0).

Example 2 (different frequency response)

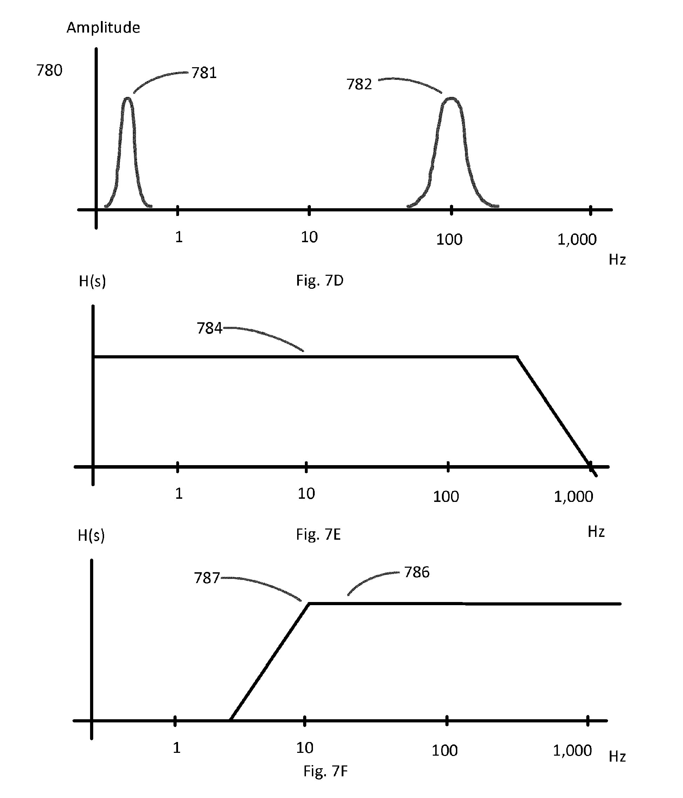

In an alternate embodiment, also schematically illustrated by Figs. 6A, 6B, and Figs. 7A-7C, the first accelerometer 301 has a low-frequency cutoff that is low enough to transduce a respiration motion. In other words, the frequency of the respiration motion is within the frequency response of the first accelerometer 301. For illustration, a plot 780 of the frequencies of a respiration motion of a patient (781) and the frequencies of the patient's gross motion (782) is schematically illustrated in Fig. 7D, and the frequency response characteristic 784 ["H(s)"] of the first accelerometer (301) is schematically illustrated in Fig. 7E. As shown in this example, the respiration frequency 781 is, in this less than 1 Hz and the gross motion frequency 782 has components about 100 Hz. The frequency response characteristic 784 of the first accelerometer (301) has a bandwidth that spans both the respiration frequency 781 and the gross motion frequency 782, so that the first accelerometer will produce an output signal in response to both the respiration motion and the gross motion of the patient.

A second accelerometer 311 has a frequency response which does not overlap with the frequency content of the respiration signal. For example, as schematically illustrated in Fig. 7F, accelerometer 311 may have a high pass frequency response (786) which will block the respiration signal. For example, the frequency response 786 of the second accelerometer 311 has a low-frequency cutoff (787) between the respiration frequency 781 and the gross motion frequency 782, such that the second accelerometer will produce an output signal in response to the the gross motion of the patient, but will filter out (i.e., dampen, or not produce, an output signal in response to) the patient's respiratory motion.

It should be noted that Vout2 does not include a response to respiration (i.e., there is no "BV_respiration2"). This is because the respiration signal is outside of the response characteristic (and specifically, outside of the frequency range or bandwidth of) the second accelerometer 311. The output signals (e.g., 5301, 5311, respectively) from the first accelerometer 301 and the second accelerometer 311 may then be processed as described above to produce a respiration signal (i.e., V_respiration_signal).

In some embodiments, it may be desirable to further process the output signals from the first accelerometer 301 and the second accelerometer 311 to reduce the impact of the noise. In some embodiments, the output signals from the first accelerometer 301 and the second accelerometer 311, or the difference between those outputs, may be digitized by an analog-to-digital converter, as schematically illustrated in Fig. 5B and 5C, for example, and provided to a digital signal processor 501 as signal data. The digital signal processor 501 may then apply a digital filter to the signal data to filter out some of the noise.

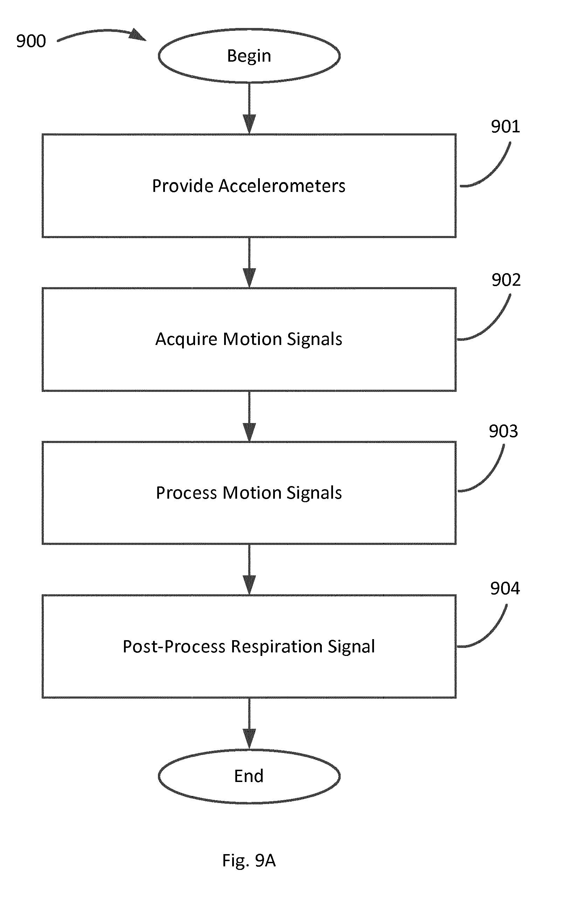

A method 900 of determining respiratory motion in a patient is schematically illustrated in a flow chart in Fig. 9A. At step 901, the method 900 provides two accelerometers, and affixes them to a patient, as described in various of the foregoing embodiments. At step 902, the method 900 then uses the accelerometers to acquire motion signals (or motion signal data) from the accelerometers, either directly or via a signal processing system (e.g., system 500) as described in various of the foregoing embodiments.

The method 900, at step 903, processes the motion signals to produce a respiration signal, as described in various of the foregoing embodiments. For example, step 903 may include calculating the net difference between the signals, as described above, and/or amplifying, buffering, analog-to-digitally converting, delaying, and/or filtering of one or more of the motion signals.

Then, in step 904, the method 900 may optionally perform some post-processing or analysis on the respiration signal. For example, the method 900 may process the data to identify peaks that represent inhaling and exhaling, and by counting the peaks, and/or the time at which the peaks occur, the method 900 may determine a respiration rate for the patient.

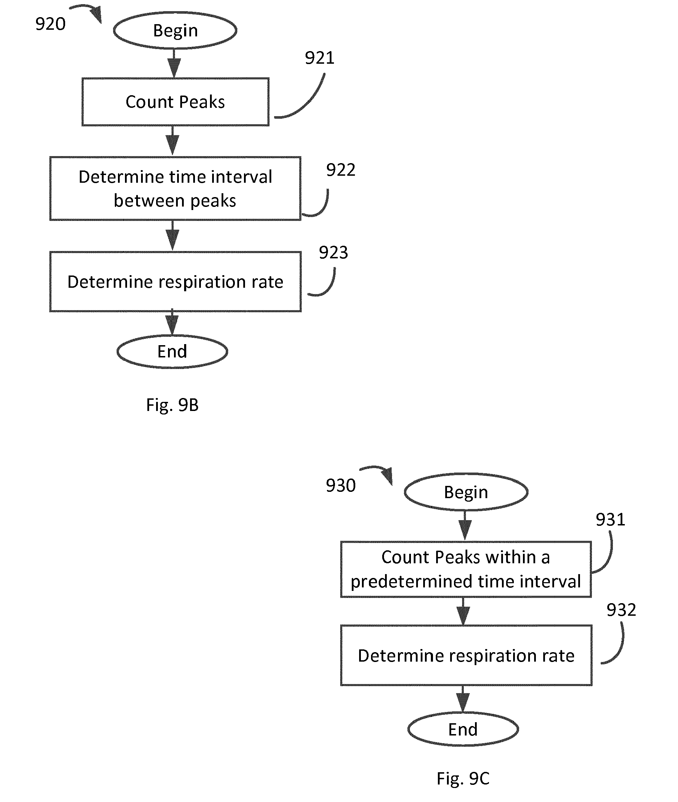

For example, a method 920 of determining a respiration rate is schematically illustrated in Fig. 9B. For example, if the respiration signal is converted from analog to digital form using an analog-to-digital converter 523, the resulting data may be processed by a digital signal processor 501. At step 921, the DSP 501 may count a pre-determined number of peaks within the data, and at step 922 the DSP 501 may determine the amount of time that passed between the first and last peak within that number of peaks. At step 923, the method 920 determines the respiration rate by dividing the number of peaks by the amount of time that passed between the first and last peak.

In an alternate embodiment schematically illustrated in Fig. 9C, a method 930 counts the number of peaks, within the data, which peaks occur over a pre-determined time interval (step 931). At step 932, the method 930 determines the respiration rate by dividing the number of peaks by the pre-determined amount of time.

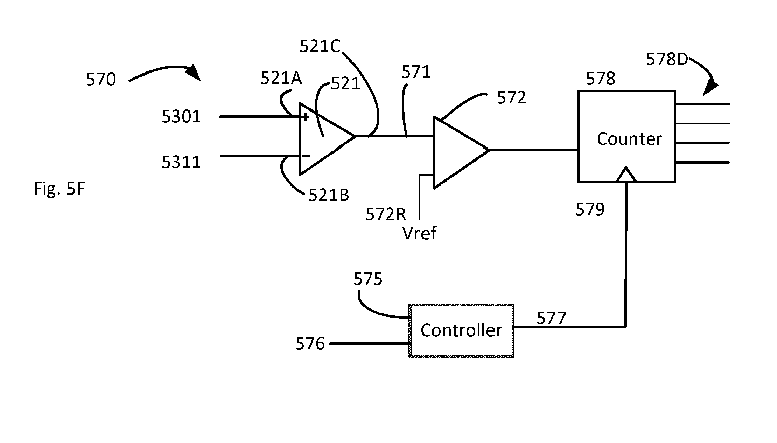

Method 930 may be implemented, in part, on a DSP 501 operating on digitized data. Alternately, method 930 may be implemented in a circuit such as circuit 570, as schematically illustrated in Fig. 5F. For example, the output 521C of amplifier 521 produces a respiration signal 571. The respiration signal is input to a comparator 572, which compares the respiration signal 571 to a reference voltage (Vref) 572R. If and when the respiration signal 571 exceeds the reference voltage 572R, the output 572C of the comparator 572 increments a counter 578. In this way, the counter counts the number of breaths drawn by the patient. The number of breaths counted is provided as a binary output at digital interface, and represents the respiration rate of the patient over the time during which the patient's breaths are counted.

The time during which breaths our counted by counter 578 may be controlled by a controller 575, and may be pre-determined. The controller may a state machine that changes state with each pulse of an input clock 576, or a timer that counts such clock pulses. Alternately, in some embodiments, the controller may be the DSP 501. The controller periodically sends a reset signal 577 to reset input 579 of controller, causing the digital output 578D to reset to binary zero. After a reset, and prior to the next reset, the counter counts the number of the patients breaths, as described above. In some embodiments, the time between resets may be the interval over which the patient's respiration rate is calculated. For example, fi the time between resets is one minute, the respiration rate of the patietnt is the number of peaks counted during that minute, and may be expressed in breaths per minute. In some embodiment, the DSP 501 may read the digital output 578D after a pre-determined amount of time, so that the DSP 501 has the data and also has information about the time interval over which the pulses were counted.

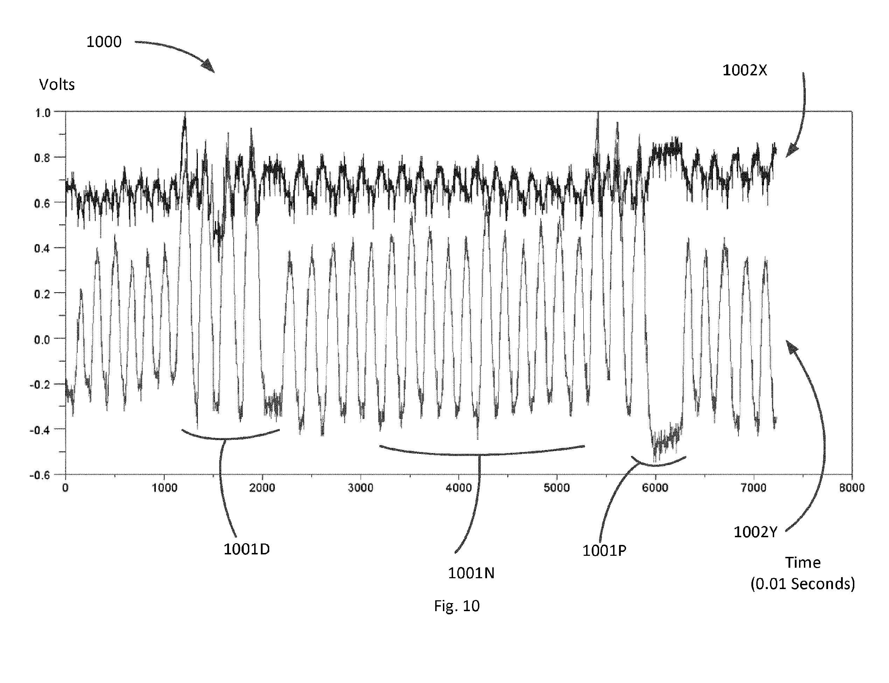

Although the foregoing embodiments have been described as employing single-axis accelerometers, that is not a limitation on all embodiments. For example, some embodiments use one or more two-axis accelerometers or three-axis accelerometers, and select, for purposes of signal processing as described above, one of the two output signals produced by such two-axis accelerometers. An example of the outputs of from each axis of a two-axis accelerometer is schematically illustrated in Fig. 10. In particular, the accelerometer used was an ADXL362 from Analog Devices, Inc. A system 500 may use either of these signals 1002X and 1002Y as the output of the accelerometer for purposes described above.

To capture the data illustrated in Fig. 10, the accelerometer 301 was fastened to a patient's abdomen by a band, and the patient was lying on a bed. The accelerometer 301 monitored the patient's breathing in two orthogonal axes (X and Y), as illustrated by trace 1002X for the X-axis motion, and trace 1002Y for the Y-axis motion. The traces 1002X and 1002Y show motion signals due to the patient breathing normally (region 1001N), during deep breathing (region 1001D), and during a pause in the patient's breathing (region 1001P).

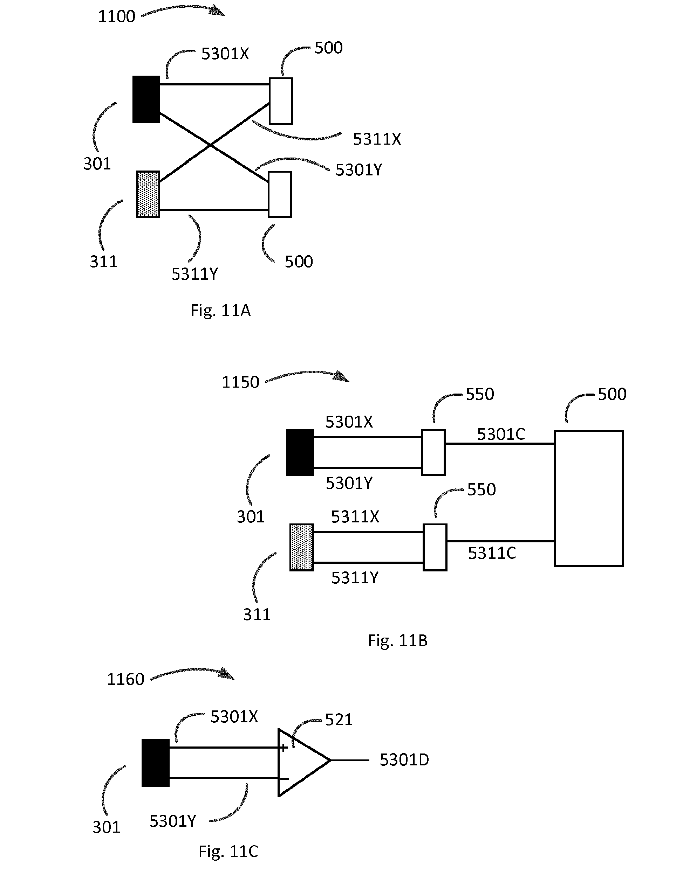

In some embodiments, signals from various two-axis accelerometers may be processed by, for example, the circuit 1100 in Fig. 11A, or circuit 1150 in Fig. 11B, to identify but a few examples.

In Fig. 11A, each of the accelerometers 301 and 311 produce two output signals (5301X and 5301Y, and 5311X and 5311Y, respectively). In this embodiment, signals 5301X and 5311X represent the respective responses of the accelerometers 301, 311 to motions in the X-axis, and signals 5301Y and 5311Y represent the respective responses of the accelerometers 301, 311 to motions in the Y-axis, where the respective X and Y axes of the accelerometer 301, 311 are aligned or at least have a common vector of sensitivity. A system may then process either the two X-axis signals (5301X and 5311X), or the two Y-axis signals (5301Y and 5311Y), or any combination of those signals, in a signal processing circuit (e.g., circuit 500).

In an alternate embodiment 1150, as schematically illustrated in Fig. 11B, the X and Y signals 5301X and 5301Y from accelerometer 301 are summed (e.g., in summing circuit 550) to produce a composite signal 5301C, and the X and Y signals 5311X and 5311Y from accelerometer 311 are summed (e.g., in summing circuit 550) to produce a composite signal 5311C. As such, each of the signals 5301X and 5301Y may be referred to as contributory signals, because they both contribute to the composite signal 5301C. Similarly, each of the signals 5311X and 5311Y may be referred to as contributory signals, because they both contribute to the composite signal 5311C.

The composite signals 5301C and 5311C may then be provided to a signal processing circuit 500 (e.g., as signal 5301 and 5311 in Figs. 5B-5E). In Fig. 11A and Figs. 11B, signal processing circuit 500 may be any of the circuits in Figs. 5A-5C, to identify but a few examples.

In an alternate embodiment 1160, as schematically illustrated in Fig. 11C, the Y signal 5301Y is subtracted from the X signal 5301X, for example by differential amplifier 521, to produce a signal representative of the patient's respiration. For example, consider an embodiment in which if the Y-axis signal 5301Y includes a component in response to an aggressor signal having an amplitude of "A," and a component in response to a patient's respiration having an amplitude of, for example. 0.25 volts. Consider also that the embodiment produces an X-axis signal 5301X that also has an a component in response to an aggressor signal having an amplitude of "A," but has a component in response to a patient's respiration having an amplitude less than the amplitude of the component in response to a patient's respiration in signal 5301Y. For example, consider that the component of signal 5301X in response to a patient's respiration has an amplitude of 0.15 volts. In this example, the result of the subtraction (e.g., the output 5301D of the differential amplifier 521) would be expressed by the following equation:

$$(5)\ \mathrm{Vout} = A + 0.25V - (A + 0.15V) =$$

$$A + 0.25V - A - 0.15V = 0.10V.$$

In other words, the result of subtracting the signal of one axis (5301Y) from the signal from another axis (5301X) is a signal of amplitude 0.1V representing only the respiration of the patient.

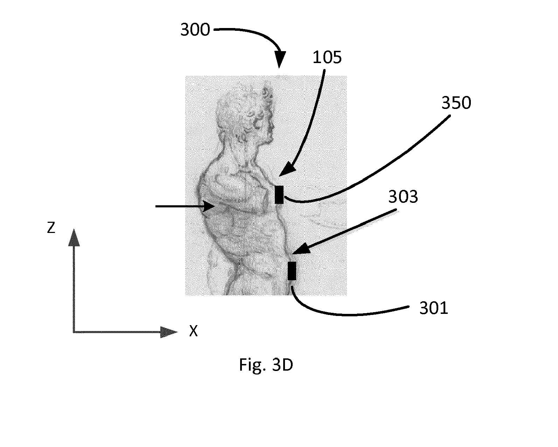

An alternate embodiment is schematically illustrated in Fig. 3D, and has a first accelerometer 350 disposed on the chest 105 of a patient 300, and a second accelerometer 301 disposed on the abdomen 330 of the patient 300. Both of the accelerometers 301, 350 sense a gross motion of the patient 300, and both accelerometers 301, 350 respond to respiratory motion of the patient 300. However, the responses of the two accelerometers 301, 350 to respiratory motion are not identical, even if the accelerometers 301, 350 are identical to each other. For example, the response of the accelerometer 350 to gross motion and respiratory motion may be described by the following equation:

$$(6)\ V350 = V(\text{gross motion}) + nV(\text{respiratory motion})$$

and the response of the accelerometer 301 to gross motion and respiratory motion may be described by the following equation:

$$(7)\ V301 = V(\text{gross motion}) + mV(\text{respiratory motion})$$

where "n" and "m" are scale factors that represent a fraction of the patient's respiratory motion that contributes to the output signals of the accelerometers V350 and V301, respectively. For example, the scale factors "m" and "n" may be determined by the sensitivity of the accelerometers 301 and 350, or by the amount of respiration signal that reaches each accelerometer 350, 301, respectively. In any case, in the foregoing embodiment, n is not, and must not be, equal to m.

From these two signals (V350; V301), the respiration signal may be extracted or produced by determining or producing the difference between them (i.e., subtracting one of the signals from the other). For example, a respiration signal may be expressed as:

14

$$(8) \quad V301 - V351 = V(\text{gross motion}) + mV(\text{respiratory motion}) - V(\text{gross motion}) - nV(\text{respiratory motion}) = (m-n)V(\text{respiratory motion}).$$

In a preferred embodiment, n = 0 and m =1, so that the resulting signal is an un-scaled version of the respiratory signal, i.e., (m-n)V(respiratory motion) = (1-0)V(respiratory motion) = V(respiratory motion).

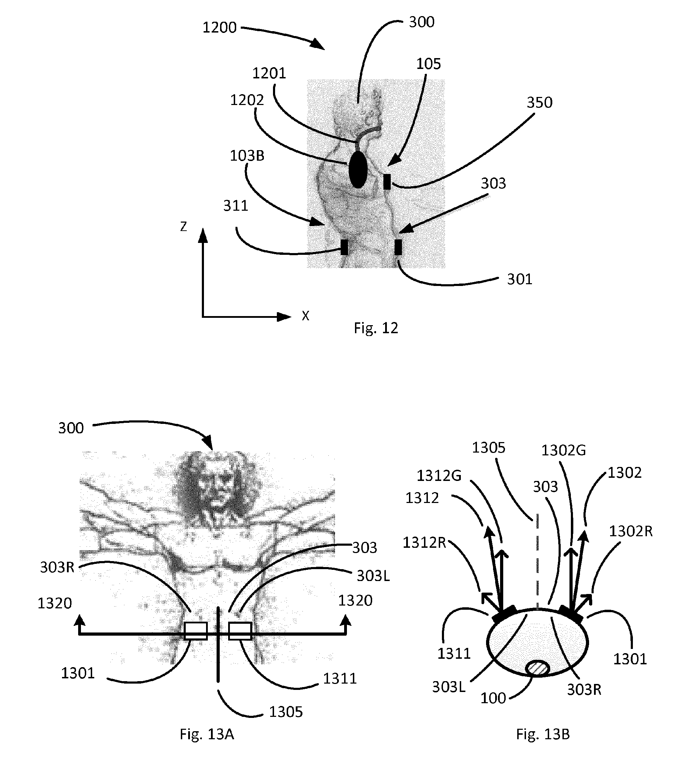

Fig. 12 schematically illustrates an alternate embodiment of a respiration sensing system 1200. System 1200 includes three accelerometers 301, 311 and 350, disposed on the lower back 103B, abdomen 303, and chest 150, respectively, of the patient 300.

The system 1200 may be used according to any of the two-accelerometer embodiments described above by, for example, processing the outputs of two of the three accelerometers (301, 311, 350). Alternately, or in addition, the system 1200 may detect abnormal respiration, such as the respiration that occurs in a patient 300 with sleep apnea, for example.

During sleep apnea, the airway 1201 to the lungs 1202 of patient 300 is blocked by the tongue. Also, during sleep apnea the chest 105 and abdomen 303 of the patient 300 move in anti-phase. In other words, when the patient's chest 105 moves outward, for example during inhalation, the patient's abdomen 303 moves inward, such that at any given moment, the patient's chest 105 and abdomen 303 move in opposite directions.

Such antiphase motion between the chest 105 and abdomen 303 may be used to detect sleep apnea. The system 1200 is configured to detect antiphase motions between the accelerometers 301 and 350 by the use of the third accelerometer 311, although detection of sleep apnea may be performed by the use of only two accelerometers - e.g., one on the chest and another on the abdomen. More specifically, in system 1200, all three accelerometers 301, 311 and 350 are configured to produce a positive voltage output signal in response to motion in the +X direction, and a negative voltage output signal in response to motion in the -X direction.

When all three accelerometers 301, 311 and 350 detect the gross motion of the patient 300, the motion of accelerometer 311, as represented by its output signal, may be subtracted from the output signals of accelerometers 301 and 350, to produce signals representing the net motions of the patient's abdomen 303 and chest 105, respectively, as described and illustrated in connection with equations (1) - (4), above.

The system 1200 may operate according to a method schematically illustrated by steps of the flow chart 900 in Fig. 9. For example, if the system 1200 includes only the accelerometers on the chest 350 and abdomen 301, then the method 900 includes steps 901-903. The method 900 begins by providing those accelerometers 350, 301 and affixing them to the patient's chest and abdomen, respectively (step 901) and using them to detect the respective motions of the patient's chest 105 and abdomen 303. As such, accelerometer 350 produces a chest motion signal at its output, and accelerometer produces an abdomen motion signal at its output.

The chest motion signal and the abdomen motion signal are processed at step 903 to determine whether the patient is experiencing sleep apnea, by determining whether the chest motion signal and the abdomen motion are in antiphase.

In a system 1200 with three accelerometers, the step 901 of providing accelerometers include providing accelerometers 350 and 301 as described above, and further includes providing accelerometer 311 and affixing accelerometer to the patient's lower back region 103B, for example such that the axis of sensitivity of the third accelerometer 311 is perpendicular to the patient's spine, and using the third accelerometer 311 to detect the gross motion of the patient 300 at step 902. Since the output signals from the first two accelerometers 350 and 301 also include responses, respectively, to gross motion, a net abdominal motion signal may be produced by subtracting the gross motion signal from the third accelerometer 311 from the abdominal motion signal from the second accelerometer 301 to produce a net abdominal motion signal, at step 903. Similarly a net chest motion signal may be produced by subtracting the gross motion signal from the third accelerometer 311 from the chest motion signal from the first accelerometer 350 to produce a net chest motion signal, also at step 903. The net chest motion signal and the net abdomen motion signal are processed at step 903 to determine whether the patient is experiencing sleep apnea, by determining whether the net chest motion and the net abdomen motion are in antiphase.

Fig. 13A and Fig. 13B schematically illustrate another embodiment in which two accelerometers 1301 and 1311 are affixed to the abdomen 303 of a patient 300. As schematically illustrated in Fig. 3A, the accelerometers 1301 and 1311 are disposed on opposite sides (i.e., the right and left sides, respectively) of the patient's abdomen. For example the accelerometers 1301 and 1311 are disposed on opposite sides of a vertical plane 1305 that bisects the patient's abdomen. Fig. 3B schematically illustrates a cross-section through the patient's abdomen 303 along line 1320, and shows the accelerometers 1301 and 1311 as well as the patient's spine 100.

In operation, during respiration of the patient 300, each of the accelerometers 1301 and 1311 experience motion, as indicated by motion vectors 1302 and 1312, respectively. Motion vector 1302, in turn, includes a vector 1302R due to respiration, and a vector 1302G due to gross motion. The accelerometer 1301 responds to both of those vectors, so that the output of accelerometer 1302 may be expressed as the following equation:

$$(9) \quad Vout1301 = V\_respiration1301 + V\_gross1301.$$

Similarly, motion vector 1312 includes a vector 1312R due to respiration, and a vector 1312G due to gross motion. The accelerometer 1311 responds to both of those vectors, so that the output of accelerometer 1311 may be expressed as the following equation:

$$(10) \quad Vout1311 = V\_respiration1311 + V\_gross1311.$$

It should be noted that, in this embodiment the respiration vectors 1302R and 1312R point in slightly opposing directions, as schematically illustrated in Fig. 13B, since they are disposed on opposite sides (303R and 303L) of the abdomen 303, and those opposite sides of the abdomen move differently in response to respiration. However, the gross motion vectors 1302G and 1312G substantially identical in magnitude and are substantially parallel.

Consequently, the respiration motion may be found by subtracting the output of one accelerometer from the output of the other:

$$Vrespiration = \quad Vout1301 - Vout1311$$

$$= V\_respiration1301 + V\_gross1301 - (V\_respiration1311 + V\_gross1311)$$

$$= V\_respiration1301 + V\_gross1301 - V\_respiration1311 - V\_gross1311$$

Such a subtraction may be performed in electronic circuitry, such as by circuits 520 in Fig. 5B, or circuit 530 in Figs. 5C, to name but a few examples. In a case where the gross motion vectors are equal to on another (i.e., V_respiration1301 = V_respiration1311), then the resulting respiration signal may be expressed as:

$$(11) \quad Vrespiration = V\_respiration1301 + V\_respiration1311.$$

Generally, the embodiments described herein are configured to determine respiratory motion by using signals from two (or more) sensors, such as accelerometers. However, other types of motion sensors may be employed. For example, respiratory motion may be detected and quantified using MEMS gyroscopes, for example. Also, various embodiments described above have been illustrated with voltage-output sensors, but other sensors could be used, such as current-output sensors for example. As such, any of the sensors described above could be either voltage-output sensors or current-output sensors.

Various embodiments of the present inventions may be characterized by the potential claims listed in the paragraphs following this paragraph (and before the actual claims provided at the end of this application). These potential claims form a part of the written description of this application. Accordingly, subject matter of the following potential claims may be presented as actual claims in later proceedings involving this application or any application claiming priority based on this application. Inclusion of such potential claims should not be construed to mean that the actual claims do not cover the subject matter of the potential claims. Thus, a decision to not present these potential claims in later proceedings should not be construed as a donation of the subject matter to the public.

Without limitation, potential subject matter that may be claimed (prefaced with the letter "P" so as to avoid confusion with the actual claims presented below) includes:

P1: A method of detecting sleep apnea in a patient, comprising:

affixing a first accelerometer to an anterior portion of the patient's lower abdominal region, the first accelerometer having a first axis of sensitivity, and configured to produce a first motion signal indicative of motion along the first axis of sensitivity;

affixing a second accelerometer to a chest region of the patient, the second accelerometer having a second axis of sensitivity substantially parallel to the first axis of sensitivity and configured to produce a second motion signal indicative of motion along the second axis of sensitivity, and

processing the first motion signal and the second motion signal to determine whether the patient is experiencing sleep apnea by determining whether the first motion signal is in antiphase with the second motion signal.

P2: The method of detecting sleep apnea in a patient according to potential claim P1, further including:

affixing a third accelerometer to the patient's lower spinal region, the third accelerometer having a third axis of sensitivity substantially perpendicular to a portion of the subject's back, and configured to produce a third motion signal indicative of motion along the third axis of sensitivity;

processing the first motion signal and the third motion signal to produce a net abdominal motion signal;

processing the second motion signal and the third motion signal to produce a chest abdominal motion signal;

processing the net abdominal motion signal and the net chest motion signal to determine whether the patient is experiencing sleep apnea by determining whether the net abdominal motion signal is in antiphase with the net chest motion signal.

P3: A method of detecting the respiration of a subject, the subject having a an abdomen, comprising:

affixing a first accelerometer to a left portion of the patient's abdomen, the first accelerometer disposed to produce a first motion signal in response to a the patient's respiration and gross motion;

affixing a second accelerometer to a right portion of the patient's abdomen, the second accelerometer disposed to produce a second motion signal in response to a the patient's respiration and gross motion; and

processing, in a circuit, the second motion signal and the first motion signal to produce a respiration signal, the respiration signal indicative of the motion of the subject's abdomen in response to the subject's respiration.

In P3, the act of processing, the second motion signal and the first motion signal to produce a respiration signal may include subtracting the first motion signal from the second motion signal.

Various embodiments of the invention may be implemented at least in part in any conventional computer programming language. For example, some embodiments may be implemented in a procedural programming language (*e.g.,* "C"), or in an object oriented programming language (*e.g.,* "C++"). Other embodiments of the invention may be implemented as preprogrammed hardware elements (e.g., application specific integrated circuits, FPGAs, and digital signal processors), or other related components.

In an alternative embodiment, the disclosed apparatus and methods may be implemented as a computer program product for use with a computer system. Such implementation may include a series of computer instructions fixed either on a tangible medium, such as a non-transient computer readable medium (e.g., a diskette, CD-ROM, ROM, or fixed disk). The series of computer instructions can embody all or part of the functionality previously described herein with respect to the system.

Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies.

Among other ways, such a computer program product may be distributed as a removable medium with accompanying printed or electronic documentation (e.g., shrink wrapped software), preloaded with a computer system (e.g., on system ROM or fixed disk), or distributed from a server or electronic bulletin board over the network (e.g., the Internet or World Wide Web). Of course, some embodiments of the invention may be implemented as a combination of both software (*e.g.,* a computer program product) and hardware. Still other embodiments of the invention are implemented as entirely hardware, or entirely software.

The embodiments of the invention described above are intended to be merely exemplary; numerous variations and modifications will be apparent to those skilled in the art. All such variations and modifications are intended to be within the scope of the present invention as defined in any appended claims.

**Claims**

1. A method of detecting the respiration of a patient, comprising:

   providing a first accelerometer (301) affixed to the subject, the first accelerometer having a first response characteristic configured to produce a first output signal in response to both a respiration motion of the patient and a gross motion of the patient;
   providing a second accelerometer (311) affixed to the subject, the second accelerometer having a second response characteristic configured to produce a second output signal in response to the gross motion of the patient, but not in response to the respiration motion of the patient;
   acquiring, using a circuit, from the first accelerometer, the first motion output signal;
   acquiring, using the circuit, from the second accelerometer, the second motion output signal; and
   determining, in the circuit, the difference between the second motion signal and the first motion signal to produce a respiration signal, the respiration signal produced in response to the subject's respiration.

2. A method of detecting the respiration of a subject, the subject having a thorax (105), an abdomen, and a spine (100) comprising a lower spinal region (103) disposed between the coccyx (102) and the thorax (103), comprising:

   affixing a first accelerometer (301) to the subject's lower spinal region, the first accelerometer having a first axis of sensitivity substantially perpendicular to a portion of the subject's back, and configured to produce a first motion signal indicative of motion along the first axis of sensitivity;
   affixing a second accelerometer (311) to an anterior portion of the subject's lower abdominal region, the second accelerometer having a second axis of sensitivity substantially parallel to the first axis of sensitivity, and configured to produce a second motion signal indicative of motion along the second axis of sensitivity, the first accelerometer and the second accelerometer disposed such that the subject's abdomen is between the first accelerometer and the second accelerometer, and the first axis of sensitivity and the second axis of sensitivity pass through the subject's abdomen; and
   processing, in a circuit, the second motion signal and the first motion signal to produce a respiration signal, the respiration signal indicative of the motion of the subject's abdomen in response to the subject's respiration.

3. The method of detecting the respiration of a patient of claim 1 or 2, further comprising:

   determining, in the circuit, the number of cycles of the respiration signal over a predefined period of time.

4. The method of detecting the respiration of a patient of claim 3, wherein the predefined period of time is one minute, such that the respiration of the patient may be expressed in term of cycles per minute.

5. The method of detecting the respiration of a patient of claim 2, wherein:

   the first accelerometer and the second accelerometer are disposed so as to produce a first motion signal that is complementary to the second motion signal in response to a commonly applied gross motion; and wherein determining the difference between the second motion signal and the first motion signal comprises summing the first motion signal and the second motion signal.

6. The method of detecting the respiration of a patient of claim 1 or any claim when dependent on claim 1, wherein providing the first accelerometer and providing the second accelerometer comprises affixing the first accelerometer and the second accelerometer to the patient such that the first accelerometer and the second accelerometer are within 3 centimeters of one another, or wherein the first accelerometer and the second accelerometer are in a stacked configuration.

7. The method of detecting the respiration of a patient of claim 1 or any claim dependent on 1, wherein:

   the first response characteristic comprises a noise floor sufficiently low such that the first accelerometer is configured to produce a first output signal in response to both a respiration motion of the patient and a gross motion of the patient; and
   the second response characteristic comprises a noise floor that is above the amplitude of the respiration motion of the patient but below the amplitude of a gross motion of the patient, such that the second accelerometer is configured to produce a second output signal in response to the gross motion of the patient, but not in response to the respiration motion of the patient.

8. The method of detecting the respiration of a patient of claim 1 or any claim dependent on 1, wherein:

the first response characteristic comprises a low-frequency cutoff at a frequency sufficiently low such that the first accelerometer is configured to produce a first output signal in response to both a respiration motion of the patient and a gross motion of the patient; and
the second response characteristic comprises a low-frequency cutoff at a frequency greater than a frequency of the patient's respiration motion, but lower than a frequency of the patient's gross motion, such that the second accelerometer is configured to produce a second output signal in response to the gross motion of the patient, but not in response to the respiration motion of the patient.

9. The method of detecting the respiration of a patient of claim 1 or any of claims 3 to 8 when dependent on claim 1, wherein:

a) the first response characteristic comprises a noise floor sufficiently low such that the first accelerometer is configured to produce a first output signal in response to both a respiration motion of the patient and a gross motion of the patient; and the second response characteristic comprises a low-frequency cutoff at a frequency greater than a frequency of the patient's respiration motion, but lower than a frequency of the patient's gross motion, such that the second accelerometer is configured to produce a second output signal in response to the gross motion of the patient, but not in response to the respiration motion of the patient, and/or
b) the first response characteristic comprises a low-frequency cutoff at a frequency sufficiently low such that the first accelerometer is configured to produce a first output signal in response to both a respiration motion of the patient and a gross motion of the patient; and the second response characteristic comprises a nose floor that is above the amplitude of the respiration motion of the patient but below the amplitude of a gross motion of the patient, such that the second accelerometer is configured to produce a second output signal in response to the gross motion of the patient, but not in response to the respiration motion of the patient.

10. The method of detecting the respiration of a patient of any preceding claim, wherein:

providing a first accelerometer comprises providing a providing a first accelerometer having a first axis of sensitivity and a third axis of sensitivity, the first accelerometer configured to produce a first contributory signal in response to motion sensed by the first accelerometer along the first axis of sensitivity, and configured to provide a third contributory signal in response to motion sensed by the first accelerometer along the third axis of sensitivity;
and wherein providing a second accelerometer comprises providing a providing a second accelerometer having a second axis of sensitivity and a fourth axis of sensitivity, the second accelerometer configured to produce a second contributory signal in response to motion sensed by the second accelerometer along the second axis of sensitivity, and configured to provide a fourth contributory signal in response to motion sensed by the second accelerometer along the fourth axis of sensitivity;
and wherein the method further comprises summing the first contributory signal and the third contributory signal to produce the first motion signal, and summing the second contributory signal and the fourth contributory signal to produce the second motion signal.

11. A system for detecting the respiration of a patient, comprising:

a first accelerometer configured to be affixed to the patient so as to be subject to a gross motion of the patent and a respiration motion of the patient, the first accelerometer having a first response characteristic configured to produce a first output signal in response to both the respiration motion of the patient and the gross motion of the patient;
a second accelerometer configured to be affixed to the patient so as to be subject to at least the gross motion of the patent, the second accelerometer having a second response characteristic configured to produce a second output signal in response to the gross motion of the patient, but not in response to the respiration motion of the patient; and
a circuit configured to determine the difference between the second motion signal and the first motion signal and to produce a respiration signal, the respiration signal produced in response to the subject's respiration.

12. The system for detecting the respiration of a patient of claim 11, wherein the second accelerometer is disposed proximate to the first accelerometer such that the first accelerometer and the second accelerometer are subject to substantially identical respiration motion.

13. The system for detecting the respiration of a patient of claim 12, wherein the second accelerometer is disposed within 3 centimeters of the first accelerometer, or wherein the first accelerometer and the second accelerometer are in a stacked configuration.

14. The system for detecting the respiration of a patient of claim 13, wherein the second accelerometer is disposed such that the second accelerometer and the first accelerometer are not subject to substantially identical respiratory motion.

15. The system for detecting the respiration of a patient of any one of claims 11 to 14, wherein:

the first accelerometer has a first axis of sensitivity and a third axis of sensitivity, the first accelerometer configured to produce a first contributory signal in response to motion sensed by the first accelerometer along the first axis of sensitivity, and configured to provide a third contributory signal in response to motion sensed by the first accelerometer along the third axis of sensitivity;

the second accelerometer has a second axis of sensitivity and a fourth axis of sensitivity, the second accelerometer configured to produce a second contributory signal in response to motion sensed by the second accelerometer along the second axis of sensitivity, and configured to provide a fourth contributory signal in response to motion sensed by the second accelerometer along the fourth axis of sensitivity; and the system further comprises:

a first summing circuit configured to receive and sum the first contributory signal and the third contributory signal to produce the first motion signal; and

a second summing circuit configured to receive and sum second contributory signal and the fourth contributory signal to produce the second motion signal.

Fig. 1A

Fig. 1B

EP 2 974 648 A1

Fig. 2A
Prior Art

Fig. 2B
Prior Art

Fig. 2C
Prior Art

Fig. 2D

Fig. 2E

Fig. 3A

Fig. 3B

Fig. 3C

Fig. 3D

Fig. 4

Fig. 5A

EP 2 974 648 A1

Fig. 5B

520

5301

5311

527
521A
521
521C
522
523
501
DSP
503
XMT
529
521B
527

Fig. 5C

530

5301

5311

526
522
523
529
F
A/D
501
DSP
503
XMT
526
522
523
529

Fig. 5D

540

5301

5311

503
XMT

Fig. 5E

550

5301

5311

521C

Fig. 5F

570

521C
521A  521
5301  +
5311  −
521B

571

572
572R
Vref

578
578D
Counter

579

575
576 — Controller — 577

Fig. 6A

Fig. 6B

Fig. 6C

Fig. 7A

Fig. 7C

Fig. 7B

Fig. 7D

Fig. 7E

Fig. 7F

Fig. 8A

Fig. 8B

900

Begin

Provide Accelerometers

901

Acquire Motion Signals

902

Process Motion Signals

903

Post-Process Respiration Signal

904

End

Fig. 9A

920

Begin

921

Count Peaks

922

Determine time interval
between peaks

923

Determine respiration rate

End

Fig. 9B

930

Begin

931

Count Peaks within a
predetermined time interval

932

Determine respiration rate

End

Fig. 9C

Fig. 10

1100

5301X          500

5311X

301

5301Y

311      5311Y      500

Fig. 11A

1150

550          500

5301X

5301C

5301Y

301

5311X          550

5311C

5311Y

311

Fig. 11B

1160

5301X          521

5301D

301      5301Y

Fig. 11C

Fig. 12

Fig. 13A

Fig. 13B

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 14 19 8066

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/066064 A1 (JANGLE JEETENDRA [US] ET AL) 17 March 2011 (2011-03-17) <br> * paragraph [0008] * <br> * paragraph [0024] - paragraph [0027]; figure 1 * <br> * paragraph [0047] * <br> * paragraph [0054] - paragraph [0056]; figure 6 * <br> * claims 1,10,11 * <br> ----- | 1,3,4, 6-15 | INV. <br> A61B5/00 <br> A61B5/08 <br> A61B5/113 |
| X | US 6 997 882 B1 (PARKER B EUGENE [US] ET AL) 14 February 2006 (2006-02-14) <br> * column 25, line 60 - column 27, line 26; figure 7 * <br> ----- | 1,10,11, 15 | |
| A | US 2006/219016 A1 (WANG LI-PENG [US] ET AL) 5 October 2006 (2006-10-05) <br> * abstract * <br> * paragraph [0018] - paragraph [0021] * <br> ----- <br> -/-- | 7-9 | |

|  | TECHNICAL FIELDS SEARCHED (IPC) |
|---|---|
|  | A61B <br> G01P |

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 17 April 2015 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**Europäisches Patentamt**
**European Patent Office**
**Office européen des brevets**

## PARTIAL EUROPEAN SEARCH REPORT

Application Number

EP 14 19 8066

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | US 2013/131525 A1 (YIN BIN [NL] ET AL) 23 May 2013 (2013-05-23) * paragraph [0016] * * paragraph [0034] - paragraph [0037] * * paragraph [0043] - paragraph [0047] * * paragraph [0065] * ----- | 1,3,6, 10-15 | |
| A | US 2012/296221 A1 (MORREN GEERT GUY GEORGES [BE]) 22 November 2012 (2012-11-22) * paragraph [0018] * ----- | 8,9 | |
| | | | TECHNICAL FIELDS SEARCHED (IPC) |

EPO FORM 1503 03.82 (P04C10)

Europäisches
Patentamt
European
Patent Office
Office européen
des brevets

Application Number

EP 14 19 8066

Claim(s) completely searchable:
        1, 3, 4, 6-15

Claim(s) not searched:
        2, 5

Reason for the limitation of the search:

The present opinion, as well as the attached search report, have been
restricted to the subject-matter indicated by the applicant in his letter
of 13-03-2015, i.e. claim 1 and its dependent claims, filed in reply to
the invitation pursuant to Rule 62a(1) EPC.

**ANNEX TO THE EUROPEAN SEARCH REPORT
ON EUROPEAN PATENT APPLICATION NO.**

EP 14 19 8066

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2015

| Patent document cited in search report | Publication date | Patent family member(s) | Publication date |
|---|---|---|---|
| US 2011066064 A1 | 17-03-2011 | NONE | |
| US 6997882 B1 | 14-02-2006 | NONE | |
| US 2006219016 A1 | 05-10-2006 | US 2006219016 A1<br>WO 2006105528 A1 | 05-10-2006<br>05-10-2006 |
| US 2013131525 A1 | 23-05-2013 | CN 103052353 A<br>EP 2600767 A1<br>JP 2013535283 A<br>RU 2013109269 A<br>US 2013131525 A1<br>WO 2012017355 A1 | 17-04-2013<br>12-06-2013<br>12-09-2013<br>10-09-2014<br>23-05-2013<br>09-02-2012 |
| US 2012296221 A1 | 22-11-2012 | CN 102753095 A<br>EP 2533692 A1<br>JP 2013519421 A<br>US 2012296221 A1<br>WO 2011098944 A1 | 24-10-2012<br>19-12-2012<br>30-05-2013<br>22-11-2012<br>18-08-2011 |

EPO FORM P0459

For more details about this annex : see Official Journal of the European Patent Office, No. 12/82